# EUROPEAN PATENT SPECIFICATION

(11) **EP 2 435 552 B1**
(45) Date of publication and mention of the grant of the patent: **17.10.2018**
(21) Application number: 10781338.8
(22) Date of filing: 28.05.2010
(51) Int. Cl.: C11D 17/00, C11D 3/20, C11D 3/43, A61K 8/49, A61Q 5/02, A61Q 1/14, A61Q 19/10

(54) **SOLVENT, SOLUTION, CLEANING COMPOSITION AND METHODS**
LÖSUNGSMITTEL, LÖSUNG, REINIGUNGSZUSAMMENSETZUNG UND VERFAHREN DAFÜR
SOLVANT, SOLUTION, COMPOSITION DE NETTOYAGE ET PROCÉDÉS

(30) Priority: 29.05.2009 US 182407 P
(43) Date of publication of application: 04.04.2012
(73) Proprietor: GFBiochemicals IP Assets B.V., 6163 JT Geleen (NL)
(72) Inventor: RIETH, Lee, R., Plymouth, MN 55447 (US); YONTZ, Dorie, J., Bloomington, MN 55438 (US); LEIBING, Cora, M., Maple Grove, MN 55311 (US); TJOSAAS, Matthew, J., Minneapolis, MN 55408 (US); MURRIN, Benjamin, Sean, Hopkins, MN 55343 (US)
(74) Representative: FRKelly
(86) International application number: PCT/US2010/036720
(87) International publication number: WO 2010/138907

(56) References cited:
- EP-A1- 0 012 543
- WO-A2-2007/062118
- CH-A- 498 828
- GB-A- 1 427 918
- JP-A- H07 228 887
- US-A- 3 855 248
- US-A- 5 482 965
- US-A- 6 130 195
- US-A1- 2004 138 090
- US-A1- 2009 124 531

## Description

This present invention relates to formulated compositions, useful as cleaning products or for other applications, which contain certain alkyl ketal ester compounds.

A large number of formulated products are produced and sold. One broad class of these products includes various types of cleaning products, which can be used in industrial, domestic or other cleaning applications.

The cleaner prior art is extensive. A large number of detergent and cleaner references discuss the various formulations that can be made as aqueous solutions, aqueous concentrates, powdered or solid formulations of cleaning compositions. A variety of applications of such cleaners that have been made include hard surface cleaners, manual and automatic dishwasher formulations, laundry formulations, pot and pan degreaser formulations, floor cleaners, glass cleaners, skin cleansers, shampoos, and the like. Such formulations tend to contain a variety of ingredients but often at a minimum contain a surfactant and may contain one or more enhancing additives or solvent components. Cleaners optionally can contain a builder, a sequestrate, a second solvent, a bleach (chlorine or oxygen), and other nonfunctional ingredients such as fragrances, dyes, etc.

Because these various types of products differ enormously, as do the conditions under which they are used, the individual products tend to be formulated specifically for the end-use application for which they are intended. There are several concerns that drive the formulation choices in each case.

Product efficacy, which can be defined simply as the ability of the product to perform its intended function(s), is of course a basic concern.

Yet another constraint is a desire in some cases to avoid having certain specific types of materials in the product. For example, many products are being reformulated to at least partially remove volatile organic chemicals, including many common organic solvents. In some products, it is necessary to exclude or minimize water. In still other products, other specific materials may need to be excluded for various reasons.

In other cases, there is a desire to replace fossil fuel-based chemicals with chemicals derived from annually renewable feedstocks.

Cost is of course always a concern.

The physical form of the product is also important in many cases. The physical form of a product may be constrained by how the product is to be used. Specific applications may require the product to be in the form of a low viscosity liquid, a thickened fluid, a cream, a paste, a gel, a powder or even a solid.

It is often the case that these formulated products contain ingredients which are immiscible in each other, or which have very limited miscibility. This is commonly seen in aqueous systems that also contain oils or other highly non- polar ingredients. This immiscibility can lead to at least two problems. One is that it is sometimes difficult to formulate immiscible materials to produce a product form that is needed for a particular application, especially when the needed product form is a low viscosity liquid or a clear liquid or gel. The second problem is that even if the correct product form can be obtained, sometimes the components will nonetheless separate into layers over time. This is a very significant problem, as products are routinely stored for periods of weeks or months before reaching the consumer and even then may not be used right away. To avoid these problems, it is often necessary to introduce various types of emulsifiers, dispersants, thickeners or co-solvents into these products. This introduces complexity, increases costs, and in some cases may defeat other goals such as the reduction or elimination of volatile organic chemicals.

US3855248 discloses compounds having the formula 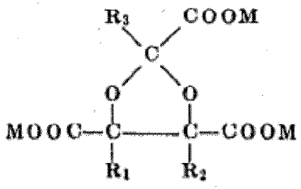 wherein R1 and R2 are hydrogen, methyl, -COOM or -CH2COOM; R3 is hydrogen, methyl or COOM; and M is alkali metal or ammonium are useful sequestrants and detergency builders. The lower alkyl ester and acid forms of such compounds are useful intermediates for their production.

US5482965 discloses method and compositions for enhancing absorption of topically administered physiologically active agents through the skin and mucous membranes of humans and animals in a transdermal device or formulation for local or systemic use, comprising a therapeutically effective amount of a pharmaceutically active agent and a non-toxic, effective amount of penetration enhancing agent of the formula I or a physiologically acceptable salt thereof: 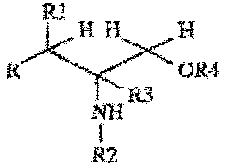 wherein: R1, R2, R3 and R4 are as defined herein.

GB1427918 discloses comprises compounds (I) and (II), 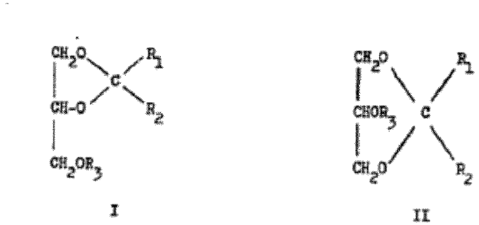 where R 1 is carboxy, alkyl, alkoxy, alkoxycarbonyl, mono-N-substituted carbamoyl, or aryl or a group - C(Y)=NOZ, where Y is alkyl or alkoxycarbonyl and Z is hydrogen, alkyl or mono-N-substituted carbamoyl; R 2 is hydrogen or alkyl; or R 1 and R 2 form a polymethylene ring; and R 3 is optionally halo-substituted aralkyl, thie preparation by reacting a diol or with a compound R 1 COR 2 or a non-cyclic acetal thereof in the presence of an acid catalyst or by reacting a compound (VI) or (VII) with R 3 halide in the presence of a strong base and, if desired, in resulting compounds, converting free carboxylic acids to amides or O-carbamoylating, O-unsubstituted oximes. The compounds of the invention are used to combat weeds at a locus and the invention includes herbicidal compositions comprising the compounds and a suitable carrier and/or surfactant.

WO2007062118 relates to the preparation of ketal compounds from glycerol and levulinic acid and esters, and uses thereof.

CH498828 discloses a process for preparing ethylene ketals of gamma-ketocarboxylic acid esters 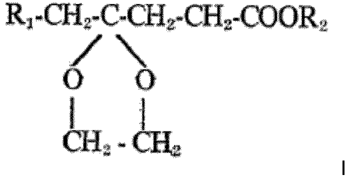 R1 = H, alkyl or aryl; R2 = lower alkyl R1-CH2-COCH2-CH2-COOR2 + CH2OH.CH2OH (I) are used as intermediates in the preparation of agricultural chemicals and medicines.

JPH07228887K discloses a composition for enhancing a refreshing feeling of menthol, improving permanence and a perfume tone of a refreshing feeling and a little irritation to scalp and skin by blending menthol with a specific dioxolan-acetic acid derivative:

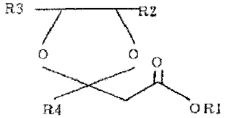

We have found that certain alkyl ketal esters falling within structure I below are excellent formulation ingredients that provide benefit in a wide number of formulated products. The alkyl ketal esters are excellent solvents for a wide range of materials. Many of them are miscible with water or other organic solvents, or both. The alkyl ketal esters described herein have low volatility. Under normal conditions of manufacture, storage, and use the alkyl ketal esters are generally unreactive with many other materials that are commonly found in formulated products.

Because of these attributes, alkyl ketal esters can be used in a wide range of cleaning compositions or formulations. In some cases, the alkyl ketal ester itself functions as a cleaning agent due to its solvency for various types of soils and greases, and can impart improved cleaning properties to a cleaner product. In other cases, the alkyl ketal esters according to structure I can function primarily as a solvent, cosolvent or emulsifier for one or more other ingredients in a product formulation. The alkyl ketal esters can in some cases function to solubilize one or more ingredients into the product formulation, or into a solvent or carrier liquid. In other cases, the alkyl ketal esters can compatibilize one or more components with other components of the product formulation, to form, for example, emulsions which can be quite storage-stable. The alkyl ketal ester(s) can in some cases allow mutually incompatible materials to be formulated into a stable form, while reducing or eliminating unwanted ingredients such as volatile organic compounds.

The alkyl ketal esters exhibit one or more of the following properties in cleaning product formulations: stability in many aqueous solutions or partially aqueous solutions; stability in many nonaqueous solutions; compatibility with chlorine or oxygen bleaches; compatibility with many formulation components commonly found in formulated products; and effectiveness in removing a variety of soils from many substrates.

A solution is disclosed of a solute in an aqueous cosolvent mixture that contains water and at least one alkyl ketal ester according to structure I below that is miscible in water at the relative proportions thereof present in the solution is described.

By "solution", it is meant a "true" solution, in which the components are uniformly mixed at a molecular level, or any other macroscopically uniform mixture in which the solute is present in the form of droplets that are smaller than the wavelength of visible light and the mixture is clear. If not molecularly dispersed, the solute should be in the form of droplets which are smaller than about 200 nm.

The term "miscible" and its variations ("miscibility", etc.) are used herein as a synonym for "soluble", as that term is defined above. A material may be fully or partially miscible in another. As used herein, a material said to be "miscible" in another, without further qualification, is miscible with that other material in all proportions, i.e., mixtures that contain only the two components form clear homogenous solutions in all weight ratios from 99.9:0.1 to 0.1:99.9. A material is said to be "highly miscible" in another material if it is soluble in the other material to the extent of at least 20 parts in 80 parts of the other material, and preferably is soluble in that other material to the extent of at least 50 parts in 50 parts of the other material. A "highly miscible" material may be soluble in the other material to the extent of 99.9 parts to 0.1 part of that other material and all lower proportions. A material is said to be "sparingly miscible" in another (or has "low miscibility" in another material) if is immiscible (as defined below) therein or otherwise not soluble in that other material to the extent of 20 parts in 80 parts of the other material. A sparingly miscible material may be soluble in that other material to the extent of no more than 10 parts in 90 parts of that other material, and may be soluble in that other material to the extent of no more than 5 parts in 95 parts of the other material. A material is said to be "immiscible" in another if it is not soluble by itself in that material to the extent of at least 1 part per 99 parts of the other material. Unless stated otherwise, miscibility is assessed at 25 °C.

By "macroscopically uniform", it is meant that the composition is uniform when viewed at a length scale of at least 10 micrometers.

In a great many cases, the cosolvent mixture includes water and the alkyl ketal ester, and the solute is at most sparingly miscible in water. In such cases, the cosolvent mixture of the invention often is a better solvent for the solute than water is by itself. This better solvency effect may allow more of the solute to be dissolved than could be in the water alone; in this manner more highly concentrated solutions of the solute can be formed than can be in just the water by itself. The improved solvency can in some instances expand the temperature range at which the solute(s) can remain dissolved. The presence of the alkyl ketal esters can, in some embodiments, improve low temperature stability or high temperature stability. This attribute can permit more latitude in the temperature range in which the solution can be stored and used. The ability to maintain a stable solution across a wide temperature range is especially important in cleaning products that contain nonionic surfactants. Nonionic surfactants tend to have become less soluble in water with increasing temperature, and therefore conventional products that contain those surfactants often exhibit a problem of becoming cloudy or even forming separate layers when the solution is heated. In other cases, surfactants can become less soluble in water with decreasing temperature, and products containing these surfactants often exhibit cloudiness or form separate layers as the solutions cool. The presence of an alkyl ketal ester often reduces the tendency of surfactant-water solutions to become cloudy or to separate upon cooling.

A solution as disclosed herein may constitute all or part of a formulated cleaning product of any of the general types described herein, or may constitute all or part of another type of product.

Additional highly water-miscible materials may be present in a solution as disclosed herein, as described more fully below. For example, water and ethanol can both be present, together with at least one alkyl ketal ester and at least one solute.

Two or more alkyl ketal esters according to structure I below can be present in a solution as disclosed herein. In certain specific embodiments, at least one highly water-miscible alkyl ketal ester is present, and at least one sparingly water-miscible alkyl ketal ester is also present.

An aqueous cosolvent mixture containing water and the alkyl ketal ester is also useful as a continuous or co-continuous phase in an emulsion. The term "emulsion" is used herein to denote a mixture of two or more immiscible liquid phases, in which (a) one of the phases is continuous and at least one of the phases is dispersed in the continuous phase or (b) the phases are co-continuous. The disperse phase should be dispersed into droplets of no larger than about 1 mm, preferably no larger than 100 micrometers in diameter. An emulsion generally is macroscopically uniform in composition. Thus, an emulsion is disclosed comprising at least one continuous phase and at least one dispersed liquid phase, or a first co-continuous phase and a second co- continuous phase, wherein at least one continuous phase or co-continuous phase includes a cosolvent mixture that includes water and an alkyl ketal ester according to structure I below.

Therefore, an emulsion is disclosed comprising at least one continuous phase that contains water and an alkyl ketal ester that is miscible in water at the relative proportions thereof present in the continuous phase.

A composition is disclosed comprising a surfactant and an alkyl ketal ester according to structure I below. The composition may take any of several forms. For example, the surfactant may be dissolved in the alkyl ketal ester or vice versa. The surfactant and alkyl ketal ester may form a single-phase mixture (i.e., a solution) together with one or more additional components, of which water is a notable example. The alkyl ketal ester may form or inhabit (i.e., reside within) a continuous phase in which the surfactant is dispersed in the form of micelles or other type of dispersed phase. The continuous phase in such a case may consist entirely of the alkyl ketal ester, or the alkyl ketal ester may form a component of a cosolvent mixture which forms all or part of the continuous phase. In another form, the alkyl ketal ester and the surfactant, optionally with other components, together form a disperse phase or a co-continuous phase of an emulsion. The alkyl ketal ester and the surfactant can form or inhabit separate, co-continuous phases in an emulsion. The surfactant can reside at least partially at the interface of a phase that contains the alkyl ketal ester and another phase. The surfactant / alkyl ketal ester composition may take the form of a solid, which may be in the form of a particulate.

These compositions may of course contain a wide number of ingredients in addition to the surfactant and the alkyl ketal ester, as may be necessary or desirable for the purposes for which the specific compositions are intended. Depending on the nature of those additional ingredients, the compositions are useful to form a wide range of formulated products, including a wide range of cleaning products as described more fully below.

This invention provides a cleaning formulation comprising from 50 to 95 weight % water, from 0.1 to 10 weight % of at least one alkyl ketal ester as defined in claim 1, and from 1 to 10 weight % of at least one surfactant. Note that throughout this specification, all percentages are by weight, and refer to weight percentages of the entire composition, unless otherwise indicated. The alkyl ketal ester suitably corresponds to structure I below, and may be a highly- water miscible type, a sparingly water- miscible type, or a mixture of both types.

The invention provides a cleaning formulation as recited in the accompanying claims, and a method for cleaning a soiled substrate, comprising bringing the composition of the invention into contact with the soiled substrate and then removing the composition cleaner together with removed soils.

A cleaning formulation is disclosed comprising 1-80 % by weight water, 1-40 weight % of an alkyl ketal ester, and from 0.1 to 10 % by weight of a surfactant. The alkyl ketal ester suitably corresponds to structure I below, and may be a highly-water miscible type, a sparingly water-miscible type, or a mixture of both types.

A cleaning composition is disclosed comprising (a) from 40 to 75 weight % water, (b) from 0.1 to 25 weight % of at least one alkyl ketal ester, c) from 0.1 to 45 weight % of at least one surfactant and at least one of component h), i), and o), wherein h), i) and o) are h) at least one builder or chelating agent; i) at least one chlorine bleach or non-chlorine bleach, and o) one or more abrasives. The alkyl ketal ester suitably corresponds to structure I below, and may be a highly-water miscible type, a sparingly water-miscible type, or a mixture of both types. In some embodiments, the cleaning composition contains up to 30% of the builder or chelating agent, up to 10% of the bleach and up to 30% of an abrasive.

A liquid laundry formulation is disclosed comprising a) 50 - 95 weight % of water, 0.1 to 25 weight % of at least one alkyl ketal ester, and from 0.1 to 45 weight % of at least one surfactant; and at least one of h), i), o), q) and r), wherein h), i), o), q) and r) are h) at least one builder or chelating agent; i) at least one chlorine bleach or non-chlorine bleach; o) one or more abrasives, q) one or more anti-deposition agents and r) one or more brightening agents. The alkyl ketal ester suitably corresponds to structure I below, and may be a highly-water miscible type, a sparingly water-miscible type, or a mixture of both types. In some embodiments, the cleaning composition of this aspect contains up to 30% of the builder or chelating agent, up to 10% of the bleach and up to 30% of an abrasive.

A powdered laundry detergent, a powdered or granulated dishwashing product or a powdered bleaching cleanser is disclosed, comprising at least one surfactant, an alkyl ketal ester, a solid phase which that includes at least one of a carrier, an abrasive, or a solid bleach, and no more than 5 weight % water; the surfactant, alkyl ketal ester and water, if any, being carried on the solid phase. The alkyl ketal ester suitably corresponds to structure I below, and may be a highly-water miscible type, a sparingly water-miscible type, or a mixture of both types.

A personal care cleaning product is disclosed comprising at least 20 % by weight water, an alkyl ketal ester having the structure I below, and one or more components selected from s) - w), wherein s) - w) are:
s) a paraffinic, naphthenic or aromatic mineral oil;
t) nonionic organic compounds which have a melting temperature of less than 45°C, have a molecular weight of at least 190, contain at least one amido, or ester group and at least one alkyl chain containing at least 8 carbon atoms, and have a solubility in water of no greater than 1 part in 99 parts of water;
u) nonionic organosilicone compounds which have a melting temperature of less than 45°C and has a solubility in water of no greater than 1 part in 99 parts of water;
v) long chain alcohols; and
w) waxes.

A cleaning composition is disclosed comprising a substantial portion of an aqueous base, a substantial effective soil removing amount of the surfactant, an effective hardness removing amount of a sequestrate, and an alkyl ketal ester. The alkyl ketal ester suitably corresponds to structure I below, and may be a highly-water miscible type, a sparingly water- miscible type, or a mixture of both types.

A dishwasher powder composition is disclosed comprising a source of alkalinity, an alkalinity stable surfactant, an alkalinity stable sequestrate and an alkyl ketal ester. The alkyl ketal ester suitably corresponds to structure I below, and may be a highly-water miscible type, a sparingly water- miscible type, or a mixture of both types.

An aqueous hand washing dish cleaner is disclosed comprising a major portion of an aqueous medium, a surfactant, a hardness sequestrate agent, a source of alkalinity, an alkyl ketal ester and a colorant or fragrance. The alkyl ketal ester suitably corresponds to structure I below, and may be a highly-water miscible type, a sparingly water-miscible type, or a mixture of both types.

An aqueous gel dishwasher formulation is disclosed comprising a source of alkalinity, an alkyl ketal ester and an aqueous alkali stable thickening agent. The alkyl ketal ester suitably corresponds to structure I below, and may be a highly-water miscible type, a sparingly water-miscible type, or a mixture of both types.

An aqueous hard surface cleaner is disclosed comprising an aqueous base, an anionic and/or nonionic surfactant and an alkyl ketal ester. The alkyl ketal ester suitably corresponds to structure I below, and may be a highly-water miscible type, a sparingly water-miscible type, or a mixture of both types.

An aqueous disinfecting hard surface cleaner is disclosed comprising an aqueous base and an organic surfactant, an organic sequestrant, an antimicrobial agent and an alkyl ketal ester. The alkyl ketal ester suitably corresponds to structure I below, and may be a highly-water miscible type, a sparingly water-miscible type, or a mixture of both types.

An aqueous pine oil containing cleaner is disclosed comprising a major proportion of water, pine oil, a surfactant, a disinfecting agent and an alkyl ketal ester. The alkyl ketal ester suitably corresponds to structure I below, and may be a highly-water miscible type, a sparingly water -miscible type, or a mixture of both types.

A powdered cleanser is disclosed comprising a major proportion of an abrasive inorganic material, a surfactant, an alkyl ketal ester and a chlorine or oxygen bleach. The alkyl ketal ester suitably has a structure corresponding to structure I below.

An aqueous glass cleaner composition is disclosed comprising a major proportion of water, a lower alkanol and an alkyl ketal ester. The alkyl ketal ester suitably corresponds to structure I below, and may be a highly-water miscible type, a sparingly water-miscible type, or a mixture of both types.

A spray-on oven cleaner is disclosed comprising a major proportion of an aqueous base, a solvent blend comprising an alkaline glycol ether solvent, an alkyl ketal ester, a source of sodium hydroxide, and an alkaline stable surfactant. The alkyl ketal ester suitably corresponds to structure I below, and may be a highly-water miscible type, a sparingly water-miscible type, or a mixture of both types.

In another aspect, the invention is a method for cleaning a soiled substrate, comprising bringing a composition of any of the foregoing aspects of the invention into contact with a soiled substrate and then removing the composition cleaner together with removed soils.

A method is disclosed for increasing the cloud point of a solution of a nonionic surfactant in water, comprising adding to the solution a highly water-miscible alkyl ketal ester of structure I below.

The alkyl ketal esters have the general formula I: 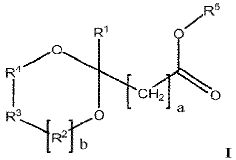 wherein a is 0 or an integer of 1 to 12; b is 0 or 1; R¹ is hydrogen, saturated or unsaturated hydrocarbon group having from having 1 to 18 carbon atoms which may be substituted with one or more heteroatoms; R², R³, and R⁴ are independently methylene, alkylmethylene wherein the alkyl group may be substituted with one or more heteroatoms, or dialkylmethylene wherein either or both of the alkyl groups may be substituted with one or more heteroatoms; and R⁵ is lower alkyl.

When b is 0, the alkyl ketal ester includes a five-membered ring; when b is 1, it includes a six-membered ring.

R¹ may be hydrogen or a linear, branched and/or cyclic hydrocarbon group. The hydrocarbon group may be saturated or unsaturated. If unsaturated, it may contain alkenyl or alkynyl groups, or both, or may contain or consist of one or more aromatic rings. R¹ may be substituted with one or more heteroatoms. In some embodiments, R¹ is preferably hydrogen or lower (C1-4) alkyl, and is more preferably hydrogen or methyl.

R² (if present) and R⁴ independently are preferably methylene, ethylidene OCH-CH₃), or 2-hydroxyethylidene (>CH-CH₂OH). R³ is preferably methylene, ethylidene, propylidene (>CH-CH₂-CH₃), >CH-CH₂OH, >C(CH₃)CH₂OH, >C(C₂H₅)CH₂OH, >C(CH₂OH)₂, >CH-CH(OH)-CH₂OH or >CH-(CHOH)₃-CH₂OH. In some embodiments, b is zero, R⁴ is methylene and R³ is one of methylene, 2-hydroxyethylidene methylene, >C(CH₃)CH₂OH or >C(C₂H₅)CH₂OH.

R⁵ preferably contains up to 4 carbon atoms, and more preferably contains 1 or 2 carbon atoms.

Preferred alkyl ketal esters include those corresponding to the reaction products of 1,2-ethylene glycol with the methyl, ethyl, n-propyl or n-butyl ester of levulinic acid; of 1,2-propylene glycol with the methyl, ethyl, n-propyl or n-butyl ester of levulinic acid; of 1,3-propane diol with the methyl, ethyl, n-propyl or n-butyl ester of levulinic acid; of glycerine with the methyl, ethyl, n-propyl or n- butyl ester of levulinic acid; of trimethylolethane with the methyl, ethyl, n-propyl or n-butyl ester of levulinic acid; of trimethylolpropane with the methyl, ethyl, n- propyl or n-butyl ester of levulinic acid; of pentaerythritol with the methyl, ethyl, n-propyl or n-butyl ester of levulinic acid; of erythritol with the methyl, ethyl, n-propyl or n-butyl ester of levulinic acid; of sorbitol with the methyl, ethyl, n-propyl or n-butyl ester of levulinic acid; of 1,2-ethylene glycol with methyl, ethyl, n-propyl or n-butyl acetoacetate; of 1,2-propylene glycol with methyl, ethyl, n-propyl or n-butyl acetoacetate; of 1,3-propane diol with methyl, ethyl, n-propyl or n-butyl acetoacetate, of glycerine with methyl, ethyl, n-propyl or n-butyl acetoacetate; of trimethylolethane with methyl, ethyl, n-propyl or n- butyl acetoacetate; of trimethylolpropane with methyl, ethyl, n-propyl or n-butyl acetoacetate, of erythritol with methyl, ethyl, n-propyl or n-butyl acetoacetate; of pentaerythritol with methyl, ethyl, n-propyl or n-butyl acetoacetate; or of sorbitol with methyl, ethyl, n-propyl or n-butyl acetoacetate.

Specific alkyl ketal esters include those having the following structures II- VI: 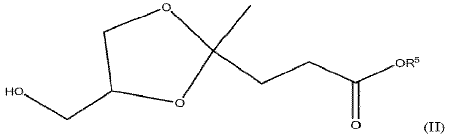 wherein R⁵ is methyl, ethyl, n-propyl or n-butyl. When R⁵ is methyl, this structure is referred to herein as "methyl-LGK" or "Me-LGK", and corresponds to the reaction product of methyl levulinate with glycerine. Methyl-LGK is miscible with water in all proportions. When R⁵ is ethyl, this structure is referred to herein as "ethyl-LGK" or "Et-LGK", and corresponds to the reaction product of ethyl levulinate with glycerine.

Ethyl-LGK is miscible in water in all proportions. Ethyl-LGK also dissolves or is miscible with a large number of hydrophobic and hydrophilic organic compounds to the extent of at least 20 parts of the organic compound in 80 parts of ethyl-LGK. Examples of such compounds include methanol, ethanol, tetrahydrofuran, acetone, ethyl acetate, ethyl laurate, lauric acid, methylene chloride, toluene, acetic acid, low molecular weight poly(propylene glycol) and castor oil. When R⁵ is n-propyl, this structure is referred to herein as "n-propyl-LGK", and corresponds to the reaction product of n-propyl levulinate with glycerine. n-propyl-LGK is miscible with water to the extent of about 1 part per 99 parts water. When R⁵ is n-butyl, this structure is referred to herein as "n-butyl-LGK", and represents the reaction product of n- butyl levulinate with glycerine. n-butyl-LGK is miscible in water to the extent of about 1 part per 99 parts of water. It dissolves or is miscible with various organic compounds to the extent of at least 20 parts of the organic compound in 80 parts of n-butyl-LGK; examples of such organic compounds include alcohols (including ethanol and 1,2-butylene glycol), organic esters (such as C12-14 alkyl benzoates, isopropyl myristate and octyl palmitate), and many vegetable oils (including castor, corn, soy and safflower oils); 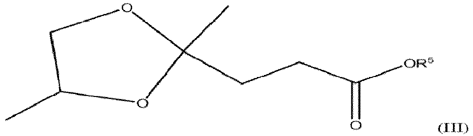 wherein R¹ is methyl, ethyl, n-propyl or n-butyl. When R⁵ is methyl, the structure is referred to herein as "methyl-LPK" or "Me-LPK" and corresponds to the reaction product of methyl levulinate with 1,2-propylene glycol. When R⁵ is ethyl, this structure is referred to herein as "ethyl-LPK" or "Et-LPK", and corresponds to the reaction product of ethyl levulinate with 1,2-propylene glycol. Ethyl-LPK is miscible in water to the extent of about 2.5 parts in 97.5 parts of water. Ethyl-LPK dissolves or is miscible with a variety of organic compounds of varying hydrophilicity, to the extent of at least 20 parts of the organic compound in 80 parts of ethyl-LPK. These compounds include, for example, methanol, ethanol, tetrahydrofuran, acetone, ethyl acetate, methylene chloride, toluene, cyclohexane, acetic acid, low molecular weight poly(propylene glycol), mineral oil, castor oil, canola oil, corn oil and sunflower oil; 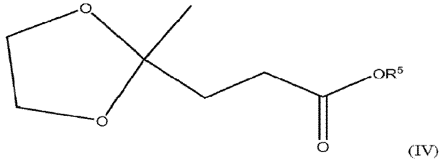 wherein R⁵ is methyl, ethyl, n-propyl or n-butyl. When R⁵ is ethyl, this structure is referred to herein as "ethyl-LEK" or "Et-LEK", and corresponds to the reaction product of ethyl levulinate with 1,2-ethylene glycol. Ethyl LEK is miscible in water to the extent of about 5 parts per 95 parts of water; 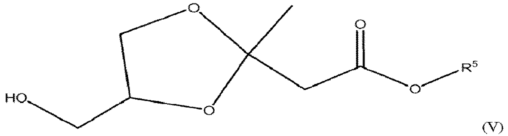 wherein R⁵ is methyl, ethyl, n-propyl or n-butyl. When R⁵ is methyl, this structure is referred to herein as "methyl-AcAcGK" or "Me-AcAcGK", and represents the reaction product of methyl acetoacetate with glycerine. When R⁵ is ethyl, this structure is referred to herein as "ethyl-AcAcGK" or "Et-AcAcGK", and represents the reaction product of ethyl acetoacetate with glycerine. Methyl AcAcGK and ethyl-AcAcGK each are miscible with water in all proportions; 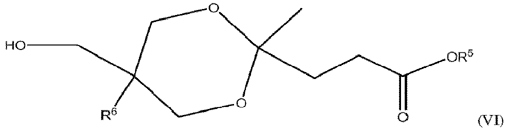 wherein R⁵ is methyl, ethyl, n-propyl or n-butyl and R⁶ is methyl or ethyl. Compounds according to structure VI correspond to the reaction product of trimethylolethane (R⁶ = methyl) or trimethylolpropane (R⁶ = ethyl) and a C 1-4 ester of levulinic acid.

The alkyl ketal esters of the present invention can be prepared by reacting an alkyl ester of an oxocarboxylate with an aliphatic polyol compound that has hydroxyl groups on adjacent aliphatic carbon atoms (as is the case with 1,2-diols) or on aliphatic carbon atoms which are separated from each other by one carbon atom (as is the case with 1,3-diols). When the starting polyol compound has hydroxyl groups on adjacent carbon atoms that react with the keto ester or semialdehyde ester, b in structure I will be zero, b in structure I will be 1 if the keto ester or semialdehyde ester reacts with hydroxyl groups on carbon atoms that are separated by another carbon atom. This reaction may be performed in the presence of an acid catalyst. A preferred process is described in WO 09/032905.

Suitable oxocarboxylates useful in making the alkyl ketal esters include both keto esters and semialdehyde esters. "Keto ester" refers to a compound having at least one ketone moiety and one carboxylic acid ester moiety. A compound may have more than one ketone functionality or more than one carboxylic acid ester functionality. "Semialdehyde ester" refers to a compound having at least one aldehyde functionality and one carboxylic acid ester functionality. A compound may have more than one aldehyde functionality or more than one carboxylic acid ester functionality. The ester group in each case is a lower alkyl group that corresponds to the R⁵ group in structure I.

The keto ester or semialdehyde ester is not particularly limited as to additional moieties or functionalities present in addition to the ketone or aldehyde and carboxylic acid ester functionalities. In some embodiments, the compound may also contain one or more halogen, carbonate, carboxylic acid, carboxylic ester, sulfone, imide, amide, amine, mercapto, protected thiol, protected hydroxyl, ether, disulfide, phosphate, phosphonoxy, siloxane, silyl, or silane functionalities. Additionally, a keto acid ester or semialdehyde acid ester may contain hydroxyl or mercapto functionality provided it is protected, e.g. by one or more trimethylsilyl or t-butyl groups, or one or more other protecting groups known to those of skill in the art.

Some examples of suitable keto esters include the Ci- C4 alkyl esters of pyruvic acid, acetoacetic acid, levulinic acid, 5-aminolevulinic acid, oxaloacetic acid, α-ketobutyric acid, α-ketoglutaric acid, α-ketoisovaleric acid, 5-ketohexanoic acid, α-ketoisocaproic acid, α-ketoadipic acid, 3-ketoadipic acid, 2-keto-4-methylthiobutyric acid, 4-acetylbutyric acid, 2-keto-3-bromobutyric acid, phenylpyruvic acid, 2-keto-3-phenylpropanoic acid, 2-ketopentanoic acid, 3- ketohexanoic acid, 4-ketohexanoic acid, 2-ketooctanoic acid, 3-ketooctanoic acid, 4-ketooctanoic acid, 7-ketooctanoic acid, 2-keto-4-pentenoic acid, 13-keto-9,II-octadecadienoic acid, 4-ketostearic acid, 9-ketopalmitic acid, 4-ketoheptanedioic acid, penicillic acid, 8-keto-8-aminopelargonic acid, 2-keto-5-aminovaleric acid, 2- succinylamino-6-oxoheptanedioic acid, 2-oxo-3-butynoate, 3-keto-6- acetamidohexanoate, and the like.

In preferred embodiments, the keto ester is a C1-C4 alkyl ester of levulinic acid (4-oxopentanoic acid). Levulinic acid is an abundant feedstock that is prepared on an industrial scale from pentoses, hexoses, and pentose- and hexose-containing polysaccharides such as cellulose, hemicellulose, starch, sucrose, and the like. Other preferred keto esters include C1-C4 alkyl esters of pyruvic acid and acetoacetic acid. Especially preferred keto esters include ethyl levulinate, n- propyl levulinate and n-butyl levulinate.

Some examples of suitable semialdehyde esters include the C1-C4 alkyl esters of aspartic semialdehyde, 4-oxobutanoic acid, 5-oxopentanoic acid, 6-oxohexanoic acid, 7-oxoheptanoic acid, α-formylglycine, 3-oxo-2-(phosphonooxy)- propanoic acid (tartronic semialdehyde wherein the hydroxyl group is protected by phosphate), 3-oxopropanoic acid (malonic semialdehyde), 2-methyl-3- oxopropanoic acid (methylmalonic semialdehyde), succinic semialdehyde, adipic semialdehyde, 5-glutamyl semialdehyde, allysine, 2-aminomuconic semialdehyde, 4-amino-5-oxopentanoic acid, N-acetylglutamic semialdehyde, 2-amino-3-(3-oxoprop-1-enyl)-but-2-enedioic acid, and N-2-succinyl-L-glutamic-5- semialdehyde. Other semialdehyde esters include the C1-C4 alkyl esters of semialdehyde compounds formed by carrying out ozonolysis of unsaturated fatty acid esters to form an aldehyde moiety at an unsaturated site, as described by Criegee, Angew. Chem. Int. Ed., 1975, 87, 745.

The polyol used to form the alkyl ketal ester may include one or more heteroatoms in addition to the hydroxyl groups. If present, these heteroatoms will form substituent groups on the R², R³ and/or R⁴ moieties in structure I. The one or more heteroatoms may be present in the form of one or more additional functional groups that can include, for example, halogen, sulfone, imide, amide, mercapto, ether, disulfide, phosphate, phosphonooxy, siloxane, silyl, or silane. The diol may contain additional hydroxyl groups or thiol groups. Suitable alkanediols include 1,2-ethanediol (ethylene glycol), 1,2-propanediol (propylene glycol), 1,3-propanediol, 2,2-dimethyl-I,3-propanediol (neopentyl glycol), 3-mercaptopropane-I,2-diol (thioglycerol), dithiothreitol, 1,2-butanediol, 1,3- butanediol, cyclohexane-I,2-diol, I,4-dioxane-2,3-diol, 3-butene-I,2-diol, indane-1,2-diol, tartaric acid, and 2,3-dihydroxyisovaleric acid. In some embodiments, alkanediols are synthesized by epoxidation of n-α-olefins such as 1-octene, 1-hexene, 1-decene, and the like, followed by ring opening to form the diol. Preferred polyol include 1,2-propanediol, 1,3- propanediol and 1,2-ethanediol, glycerine, trimethylolethane, trimethylolpropane, erythritol, pentaerythritol and sorbitol.

Certain aspects include cosolvent mixtures that contain water and at least one alkyl ketal ester. The cosolvent mixtures should be, in the absence of other materials, clear, homogeneous solutions. At least one alkyl ketal ester present in the cosolvent mixture should be miscible by itself in water at the relative proportions of water and alkyl ketal ester present in the solution. Preferably, the cosolvent mixture contains at least one highly water- miscible alkyl ketal ester, as defined before. The cosolvent mixture may contain two or more alkyl ketal esters, in which case at least one of them should be miscible by itself with water at the relative proportions of water and alkyl ketal ester that are present. In some embodiments, both a highly water-miscible and a sparingly water-miscible alkyl ketal ester are present in the cosolvent mixture.

In some embodiments, the cosolvent mixture contains at least 5 weight percent water, based on the combined weight of water, the alkyl ketal ester(s), and any additional highly water-miscible solvent(s) as may be present. In other embodiments, the cosolvent mixture may contain at least 10 weight percent water, at least 25 weight percent water, or at least 50 weight percent water, on the same basis. In some embodiments, the cosolvent mixture may contain as much as 99 weight percent water, or as much as 75 weight percent water, again on the same basis.

Additional highly water-miscible solvent(s) may be present in such a cosolvent mixture. These water-miscible solvent(s) should have a molecular weight of 250 or less, preferably 150 or less and more preferably 120 or less. Those having molecular weights of 120 or less are considered to be "volatile organic compounds" for purposes of this invention. Examples of such additional highly miscible solvents include, for example, lower alcohols such as methanol, ethanol, n-propanol and isopropanol; glycols and glycol ethers such as ethylene glycol, diethylene glycol, triethylene glycol, 1,2-propylene glycol, 1,3-propanediol, ethylene glycol monobutyl ether, diethylene glycol monomethyl ether, diethylene glycol monobutyl ether, diethylene glycol monoethyl ether, propylene glycol monomethyl ether, dipropylene glycol monomethyl ether, propylene glycol monopropyl ether; aldehydes and ketones such as formaldehyde, acetaldehyde, acetone and methyl ethyl ketone; esters such as ethyl lactate, certain chlorinated solvents, as well as other organic compounds like dimethyl isosorbide that are highly miscible by themselves in water.

Cosolvent mixtures according to one aspect of the invention are excellent solvents for a wide range of materials, including a wide range of organic materials that are sparingly miscible in water. An advantage of this aspect of the invention is that solutions of many sparingly soluble organic materials can be formed in an aqueous-based solvent system that contains little or no volatile organic chemicals, and in particular organic chemicals having a molecular weight of less than 100. The concentration of the solute in the cosolvent system is often significantly higher than can be achieved in water alone.

A wide range of materials can form the solute in this aspect of the invention. The solute in some embodiments is or includes an organic compound. Organic compounds of particular interest as the solute are those which have no or very limited miscibility with water, as the cosolvent mixture often will be a better solvent for such organic compounds than is water alone. In many cases, the solute has a solubility of no more than 5 parts per 95 parts of water, no more than 2 parts per 98 parts of water, or no more than 1 part per 99 parts water. In some embodiments, the solute may comprise more than one organic compound.

The solute must have some solubility in at least one alkyl ketal ester that is present in the cosolvent mixture. It is preferably soluble by itself to the extent of at least 10 parts by weight per 90 parts by weight of at least one alkyl ketal ester in the cosolvent mixture. More preferably, the solute is soluble to the extent of at least 20 parts per 80 parts by weight of at least one alkyl ketal ester in the solvent mixture. It may be miscible with the alkyl ketal ester to the extent of at least 50 parts per 50 parts of the alkyl ketal ester, or to the extent of 80 parts per 20 parts of the alkyl ketal ester, or miscible with the alkyl ketal ester in all proportions.

One class of solute is sparingly soluble alkyl ketal ester(s), particularly a sparingly soluble alkyl ketal ester that is miscible in water to the extent of no more than 5 parts per 95 parts water. Thus, in some embodiment, the solution of the invention is a solution that includes water, a highly water-miscible alkyl ketal ester, and a sparingly water-soluble alkyl ketal ester. In these embodiments, the sparingly water-soluble alkyl ketal ester may be present in a concentration higher than its solubility limit in water alone. The sparingly miscible alkyl ketal ester may be a functional component of the composition, for example, as a cleaning agent that provides a specific cleaning function, such as degreasing. The sparingly miscible alkyl ketal ester may also help to solubilize or emulsify yet another component which is not soluble or easily dispersible in either water or the first, water-miscible alkyl ketal ester.

Another class of solute is a surfactant, which may be of the nonionic, cationic, anionic or amphoteric type. Surfactants useful herein include well-known synthetic anionic, nonionic, amphoteric and zwitterionic surfactants. A detailed listing of suitable surfactants can be found McCutcheon's EMULSIFIERS AND DETERGENTS, North American Edition, 1984, McCutcheon Division, MC Publishing Company. Typical of these are the alkyl benzene sulfonates, alkyl-and alkylether sulfates, paraffin sulfonates, olefin sulfonates, alkoxylated (especially ethoxylated) alcohols and alkyl phenols, alkyl polygluco sides, isethionates, amides of long chain fatty acids such as the various cocoamide, lauramide surfactants; various tautrates, amine oxides, alpha -sulfonates of fatty acids and of fatty acid esters, alkyl betaines, fluorohydrocarbon surfactants (especially anionic surfactants), sulfo succinate salts and the like, which are well-known from the detergency art. In general, such detersive surfactants contain a C9-18 alkyl group. The anionic detersive surfactants can be used in the form of their sodium, potassium or triethanolammonium salts; the nonionics generally contain from about 5 to about 17 ethylene oxide groups. C 11-16 alkyl benzene sulfonates, C 12-18 paraffin-sulfonates and alkyl sulfates, alkyl polygluco sides, and the ethoxylated alcohols and alkyl phenols are especially preferred in the compositions of the present type. If the surfactant is present as a solute, its concentration will be below the critical micelle concentration of the surfactant in the particular cosolvent mixture. An advantage of the invention, when a surfactant is the solute, is that higher concentrations of the surfactant often can be dissolved in the cosolvent mixture than can be dissolved in water alone.

Other types of sparingly water-soluble organic compounds that can serve as the solute include, but not limited to, linear, branched or cyclic aliphatic hydrocarbons having up to about 30 carbon atoms; alpha-olefins having from 2 to 20 carbon atoms and which may have a boiling point of at least 100°C; cycloaliphatic hydrocarbons such as cyclopentane, cyclohexane, cyclobutane, cyclopentadiene, and the like; aromatic compounds such as benzene, naphthalene and the like; mono- or polyalkyl benzenes in which each of the alkyl group(s) contains from 1 to 12 carbon atoms, preferably 6 to 9 carbon atoms; chlorinated alkanes such as methylene chloride, chloroform, 1,2-dichloroethane, 1,1- dichloroethane; ethyl chloride and the like; brominated alkanes such as chlorobromomethane, methylene bromide and bromoform and the like; and esters such as amyl acetate and the like.

Other sparingly water-soluble organic compounds that can serve as the solute include various terpenes and various natural and synthetic oils including, but not limited to, vegetable oils, animal fats, mineral oil, silicone oils, and natural oil derivatives like fatty acid methyl esters and the like.

Various glycol ethers and glycol ether acetates that are sparingly soluble in water are also useful as the solute, including, but not limited to, dipropylene glycol monobutyl ether, dipropylene glycol monohexyl ether, propylene glycol monobutyl ether, diethylene glycol monohexyl ether, ethylene glycol n-butyl ether acetate and the like.

The solute may also include one or more one or more esters, ketones, or amides, one or more perfumes or dyes; materials such as α-glucose pyranose compounds; or enzymes such as proteases and amylases.

In the solutions the solute is present in an amount at or below its solubility limit in the cosolvent mixture, such that the combination of cosolvent mixture and solute forms a clear solution. In some embodiments, the concentration of the solute is greater than the concentration in which it can be dissolved by itself in water. Other ingredients, which are not dissolved in the solution of the invention, may cause a formulation containing the solution to be cloudy or form an emulsion.

In other aspects a cosolvent mixture containing water and a water-miscible alkyl ketal ester form a continuous or co-continuous phase in an emulsion. The physical form of such an emulsion can range from a slightly cloudy, low viscosity fluid to a highly opaque, pasty semi-solid material, depending on the particular components present in the emulsion and the relative proportions thereof. The dispersed phase or second co-continuous phase is immiscible with the cosolvent mixture that forms the continuous or other co-continuous phase at the proportions that are present. In these emulsions, the alkyl ketal esters often provide for some compatibilization between the phases, which can inhibit separation into layers and in that way form a more highly stable emulsion. Better emulsion stability can be manifested by a greater resistance to separate into layers, and/or by the ability of the disperse or co-continuous phases to resist separating into layers over a wider range of temperatures, compared to when the alkyl ketal ester is not present. The alkyl ketal ester in some cases will partition between continuous and disperse phases, or between the co-continuous phases.

The disperse or second co-continuous phase may be one or more organic materials or mixture or organic materials which is immiscible with the cosolvent mixture which forms the continuous or first co-continuous phase, or may be an organic material or mixture thereof which is somewhat miscible (sparingly soluble) in the cosolvent mixture, but nonetheless is present in an amount that exceeds its miscibility limit in the cosolvent mixture.

Therefore, any of the potential solute materials mentioned above, which have less than full miscibility in the cosolvent mixture, can form all or part of the disperse or co-continuous phase of an emulsion of the invention.

A composition is disclosed comprising at least one alkyl ketal ester of structure I and at least one surfactant. As mentioned above, a composition may take any of several forms, including various solutions and emulsions. Compositions often find use in a variety of formulated cleaning compositions. In cleaning compositions, these surfactants can provide the usual cleaning and emulsifying benefits associated with the use of such materials. Surfactants useful herein include anionic, nonionic, amphoteric and zwitterionic surfactants as described herein.

The surfactant can comprise as little as 0.1 % of a composition, preferably 1% to 10% of the surfactant. In some embodiments, the composition containing the surfactant and alkyl ketal ester may take the form of a concentrated formulation, which is designed to be diluted for use. Such a concentrated formulation typically contains no more than 60% combined of water and/or volatile organic compound, and more typically contains no more than 40% thereof. Such a concentrated formulation often contains from 5 to 10 % by weight of at least one alkyl ketal ester and from 5 to 70% or from 10 to 50% of at least one surfactant. The alkyl ketal ester in such a concentrated formulation preferably includes at least one highly water-miscible alkyl ketal ester, or a blend of at least one highly water- miscible alkyl ketal ester and a sparingly water-miscible alkyl ketal ester, if the product is designed to be dilutable in water. However, the concentrated product may contain only a sparingly water-miscible alkyl ketal ester. The surfactant/alkyl ketal ester compositions of the present invention may be formulated into clear liquids, emulsions of various types, as well as into solid forms such as granules, tablets or bars. The compositions may also be incorporated into absorbent materials such as wipes, brushes and sponges. Cleaning compositions of the invention often will contain various additional ingredients (in addition to the surfactant and alkyl ketal ester), to perform specific functions for specialized cleaning applications. Some examples of such ingredients include, but are not limited to, for example, water, detergency builders and/or chelating agents, chlorine bleaches, non-chlorine bleaches, bleach activators, bleach stabilizers, soil suspending agents, hydrotropes, highly water- miscible organic solvents as described above, sparingly water-miscible organic solvents, oils and oil derivatives, abrasives, antimicrobials, colorants, inorganic salts, thickeners, enzymes, propellants, pH control agents, fabric softening agents, additives such as metal peroxides and emollients, and the like.

A detergency builder is commonly present in laundry detergents, hard surface cleaner products and dishwashing liquids, and may be present in cleaning compositions of this invention. Examples of such builders include N-diethyleneglycol-N,N-diacetic (DIDA) acid polyphosphates (e.g., potassium pyrophosphate), nitrilotriacetates (e.g., Na3NTA), sodium ethylenediaminetetraacetate (EDTA), sodium ethylenetriaminepentaacetate, sodium citrate, sodium carbonate, sodium metasilicate and zeolites, e.g., zeolites having a cation exchange capacity (measured as CaCO₃) of 200 mg or greater per gram of zeolite. Enzymes such as proteases and amylases are also frequently present in cleaner compositions, especially laundry detergent products and prewash products.

A cleaning composition of the invention may contain a bleach such as sodium hypochlorite, sodium perborate, diperoxydodecanedioic acid, sodium dichloroisocyanurate, m-chloroperoxybenzoic acid and peroxide based bleaches. An advantage of this invention is that the alkyl ketal esters are stable in bleach solutions, and thus can add good solvency for oily soils into a bleach-containing composition. A bleach-containing composition of the invention may also contain one or more bleach activators such as tetra acetyl ethylene diamine and sodium nonanoyloxybenzene sulfonate;

A cleaning composition of the invention may further contain one or more soil suspending agents such as sodium carboxymethyl cellulose; one or more bleach stabilizers such as sodium diethylenetriamine-pentamethylenephosphonate and sodium diethyl enetriaminopentaacetate; one or more hydrotropes such as sodium toluene sulfonate, sodium cumene sulfonate and potassium xylene sulfonate; one or more fabric softening ingredients such as smectite clay and tallowdimethylammonium chloride.

A cleaning composition of the invention may contain a highly miscible organic solvent as described before.

A cleaning composition of the invention may contain one or more sparingly miscible organic solvents, including a so-called "degreasing" solvent of the type that is often incorporated into a cleaner to remove oily soils. Often, the alkyl ketal ester can replace those organic solvents in cleaner formulations, or reduce the amount thereof that is needed. An advantage of the invention, in certain embodiments, is that the alkyl ketal ester often helps to solubilize or disperse such materials into an aqueous phase. This can allow greater amounts of such solvents to be incorporated into a cleaner formulation while maintaining stability against phase separation; in other embodiments greater solution or emulsion stability can be obtained in such a solution or emulsion, at a given concentration of the organic solvent.

Examples of these sparingly miscible organic solvents include certain alcohols such as benzyl alcohol, n-hexanol, 2,2,4-trimethyl-I,3-pentanediol and terpeneoids such as menthol, terpineol, borneol, geraniol. Further examples include various sparingly water-soluble glycol ethers such as dipropyleneglycol monobutyl ether, monopropyleneglycol monobutyl ether, diethyleneglycol monohexyl ether, monoethyleneglycol monohexyl ether, butoxy propanol, butoxy propoxy propanol, diethyleneglycol monobutyl ether and mixtures thereof. Hydrocarbons or halogenated hydrocarbons of the alkyl or cycloalkyl type, and have a boiling point well above room temperature, i. e., above about 20°C are commonly used degreasing solvents which may be present in a cleaning composition of the invention. Among these are the terpenes and terpene oxides such as dipentene, pinene, d-limonene, 1-limonene, limonene oxide, myrcene and the like. Additional compounds having low miscibility in water that may be present include the Cβ-9 alkyl aromatic solvents, especially the Cβ-9 alkyl benzenes, preferably octyl benzene. These tend to exhibit excellent grease removal properties and have a low, pleasant odor.

Olefins having a boiling point of at least about 100° C, especially alpha-olefins such as 1-decene or 1-dodecene, are also excellent grease removal solvents and can be present.

Oils, both natural and synthetic, and derivatives thereof, may be present in a cleaning composition of the invention. These may function as degreasers, as solvents for specific types of soils, as rheology modifiers, as lubricants and in some cases to impart a pleasant fragrance to the composition. Such oils may include, but not limited to, mineral oil, and various types of vegetable and plant oils, including both natural and modified vegetable oils. Pine oil is a prominent example of a plant oil useful in cleaner compositions. In some embodiments, oil derivatives such as esters of fatty acids may be used in a cleaning composition.

A cleaning composition of the invention may contain one or more abrasives such as, but not limited to, silica, pumice, calcium carbonate, polyvinylchloride and perlite. It may contain other dissolved inorganic salts, such as sodium sulfate, sodium chloride and the like, which can function as viscosity modifiers, flow control agents or pH buffers. It may contain aesthetic- enhancing ingredients such as colorants and perfumes, as well as various fillers. Sodium and potassium soaps, especially coconut soaps, can be included, especially for use in creams.

In addition to the foregoing, the compositions of the invention may contain other additives such as metal peroxides and emollients.

The presence of the alkyl ketal ester can lend several advantages to a cleaning composition. The alkyl ketal esters have low volatilities and are noncorrosive. In addition, cleaning compositions in accordance with the invention can be formulated to be effective against a wide variety of soils and/or stains, and for use on a wide variety of substrates. The alkyl ketal esters often perform a valuable solubilizing and compatibilizing function, leading to cleaning compositions that have excellent resistant to phase separation. The alkyl ketal ester can also reduce the quantities of other ingredients, such as surfactants and other solvents, that are needed in the formulation. In some cases, one or more of these other ingredients can be eliminated entirely.

One class of cleaning compositions of the invention includes hard surface cleaners, which can be utilized for industrial, institutional, office or, home use. These may be formulated as, for example, general purposes hard surface cleaners, toilet cleaners, shower/bath/tile cleaners, disinfectants, soap scum removers, mildew removers, glass/mirror cleaners or stain removers. Many of these cleaners are formulated as dilute solutions or emulsions, and many are applied by spraying.

A hard surface cleaner according to the invention can include water, which will in most cases constitute at least 20 % by weight of the total composition. In some embodiments, water can constitute at least 50 % by weight of the total composition and may constitute as much as 99 % by weight of the total composition.

In some embodiments, the hard surface cleaner further includes a highly water-miscible alkyl ketal ester, preferably one that is miscible in water in all proportions. Examples of such highly water-miscible alkyl ketal esters include Me-LGK, MeAcAcGK, Et-AcAcGK or, especially Et-LGK, although mixtures of two or more of alkyl ketal esters can be used. In some embodiments, the highly water-miscible alkyl ketal ester(s) may be present in conjunction with a sparingly water-miscible alkyl ketal ester. Examples of a sparingly water- miscible alkyl ketal ester include n-propyl-LGK, n-butyl-LGK, ethyl-LPK and ethyl-LEK. In some embodiments, the alkyl ketal ester(s) may be present in an amount of up to 50 % by weight of the hard surface cleaner. The amount of alkyl ketal ester present in a hard surface cleaner composition may be from about 1 % to about 50 % by weight of the total composition, preferably from about 1 % to about 20 % by weight of the total composition, more preferably from about 3 % to about 15 % by weight of the total composition, or from about 2 % to about 10 % by weight of the total composition.

In some embodiments, hard surface cleaners may include only water and the alkyl ketal ester(s) described herein. In some embodiments the hard surface cleaner may further comprise a fragrance and/or a colorant. In these instances, the function of the alkyl ketal ester may be that of a soil remover, or, more generally, as the active cleaning agent. More typically, the hard surface cleaner will contain additional functional materials, which in most cases will include at least one surfactant. In hard surface cleaners that contain both the alkyl ketal ester and the surfactant, the alkyl ketal ester(s) may perform any or all of several functions, such as (a) solubilizing or emulsifying the surfactant and (b) functioning as an active cleaning agent. The surfactant in these hard surface cleaners is often an anionic surfactant, or a mixture of one or more anionic surfactants with one or more nonionic surfactants. Alternatively (or in addition), the surfactant may include one or more materials derived from plant sources, such as one or more Cio-iβ alkyl glycosides, an alkyl betaine, or a sulfosuccinate salt.

According to the invention, there is provided a hard surface cleaner formulation comprising, as percentages of the total formulation weight:
a) water: 50-95 %;
b-1) one or more highly water-miscible miscible alkyl ketal ester(s): 0 - 25 %, more typically 1-10 %, the alkyl ketal ester preferably including at least one of Me-LGK, Me-AcAcGK, Et-AcAcGK or, Et-LGK;
b-2) one or more sparingly miscible alkyl ketal ester(s): 0-10 %, more typically 0 to 5 %, this alkyl ketal ester preferably including at least one of n- propyl-LGK, n-butyl-LGK, ethyl-LPK and ethyl-LEK, provided that at least one of b) and c) is present;
c) one or more surfactant(s): 0.1-10 %, more preferably 0.25-10 %, still more preferably 1-7 % and in some cases 1-5 %; the surfactant(s) preferably being (1) at least one anionic surfactant, (2) at least one nonionic surfactant, (3) a mixture of at least one nonionic surfactant and at least one anionic surfactant or nonionic surfactant, (4) one or more of a Cio-iβ alkyl glycoside, an alkyl betaine or a sulfosuccinate salt, or (4) a mixture of (4) with (1), (2) or (3);
d) one or more propellants, which are generally low-boiling hydrocarbons such as butane, pentane, hexane and cyclohexane or chlorinated and/or fluorinated hydrocarbons that have boiling temperatures of from -10 °C to 50 °C;
e) one or more sparingly water-miscible organic solvent(s): generally from 0-10 %, preferably, if present, 0.1-5 %. This may be, for example, one or more of the long-chain alcohols, glycol ethers, hydrocarbons, halogenated hydrocarbons, Cβ-9 alkyl aromatic compounds, olefins, terpenes, terpene oxides, terpenoids, oils, and natural oil derivatives as described above;
f) one or more highly water-miscible organic solvents, such as a lower alcohol (e.g., ethanol andl-propanol), acetone, glycols, and glycol ethers: 0-10 %, preferably, if present, 0.1-5 %;
g) one or more antimicrobials, 0-5 %, preferably, if present 0.1-2 %, some examples of include quaternary ammonium chlorides such as C12-16 alkyl dimethyl benzyl ammonium chloride and various phenylphenol compounds;
h) one or more builders or chelating agents, particularly a chelating agent such as a phosphate salt, a citrate salt, ETDA, or DIDA: 0-30 %, preferably, if present, 0.05-10 % and more preferably, if present, from 0.5-1 %, still more preferably, if present, from 2-8 % and even more preferably, if present, from 2-6 %;
i) one or more bleaches, including, for example, a chlorine bleach such as sodium hypochlorite or a non-chlorine bleach such as hydrogen peroxide or other peroxy compound: 0-10 %, preferably, if present 0.1-5%;
j) one or more pH control agent(s) (e.g., acids, bases, pH buffers): 0-2 %, if present preferably 0.05 to 1 %;
k) one or more colorants: 0-5 %, preferably if present 0.1-2 %;
l) one or more inorganic water-soluble salts such as sodium sulfate: 0-10 %, preferably if present 0.1-5 %;
m) one or more viscosity thickeners, including, for example a water- soluble polymer: 0-10 %, preferably if present 0.1-5 %;
n) one or more proteolytic enzymes, 0-5 %: preferably if present 0.1-1 %
o) one or more abrasives; and
p) one or more fragrances.

Components b-2) and d)-p) as described above may be present in any combination of any two or more of them. Any or all of components b-2) and e) - n) may be omitted in any particular hard surface cleaner formulation, provided that if component b-2) is absent, then some of component b-1) must be present. Component d) is typically present in a spray cleaner formulation. It is noted earlier that in some instances, a single material may perform multiple functions in a hard cleaner formulation.

Component j) is often present to provide for a pH of from 3.5 or higher, preferably from 6 to 10. Some exemplary hard cleaner formulations in accordance with the invention follow. The indicated percentages are weight percents based on the total formulation weight. In these hard cleaner formulations, it is preferred that the alkyl ketal ester include at least one component b-1) material, such as, for example, ethyl-LGK, methyl-LGK, methyl-AcAcGK and ethyl-AcAcGK. These hard cleaner formulations may contain one or more component b-1) materials, one or more component b-2) materials, examples of which include methyl-LPK, ethyl-LPK, ethyl LEK, n-propyl-LGK, n-butyl-LGK, and the like, or a mixture of b-1) and b-2 materials. The functions of the alkyl ketal esters in these formulations, depending on the particular formulation, may include one or more of (1) soil dissolution and/or removal; (2) compatibilization of ingredients, particularly sparingly water-soluble ingredients into water; (3) formation of a cosolvent mixture in which one or more other ingredients are dissolved or dispersed, (4) elimination or reduction of surfactants and/or organic solvents or others.

The General Formulations 1-26 are partly outside the claimed range. The General Formulations 27-29, 31, 35-36 are outside the claimed range.

### General Formulation 1 - Aqueous Tile Shower Cleaner

| | |
|---|---|
| Anionic detergent | 0-10 % |
| Alkyl ketal ester | 1-10 % |
| Propylene glycol butyl ether | 0-10 % |
| D-Glucopyranose, oligomeric, decyl octyl glycosides | 0-10 % |
| D-Glucopyranose, oligomeric, C₁₀₋₁₆-alkyl glycosides | 0-10 % |
| Builder(s) | 0-5% |
| Antimicrobial (sodium orthophenyl phenol) | 0-5 %, preferably 0.1-5 % |
| Water | (balance of the formulation) |

At least one of the surfactants (anionic surfactant and/or one of the alkyl glycosides) is present in the above formulation. Total surfactant levels are generally in the range of 1-10 wt. %.

### General Formulation 2 - Aqueous Pine Cleaner

| | |
|---|---|
| Lower Alkanol | 0-10 % |
| Alkyl ketal ester | 0.1-25 % |
| Pine Oil | 0.1-10 % |
| Disinfectant (e.g., quaternary alkyl (C₁₂₋₁₆) dimethyl benzylammonium chloride) | 0-5, preferably 1-5 % |
| Nonionic and/or amphoteric surfactant | 1-15, preferably 1-5 % |
| Water | (balance of the formulation) |

The alkyl ketal ester and water are believed to form a cosolvent mixture in this formulation in which the lower alkanol (if present in the formulation), pine oil and surfactant are either dissolved or dispersed. The presence of the alkyl ketal ester can allow the lower alkanol to be reduced or eliminated, and the amount of pine oil to be reduced or eliminated.

### General Formulation 3 - Aqueous Soap Scum Remover

| | |
|---|---|
| Surfactant | 1-10 % |
| Diethylene Glycol Monobutyl Ether | 0-10 % |
| Chelating agent (e.g. EDTA tetrapotassium salt) | 1-10 % |
| Builder (e.g., sodium carbonate) | 0-2% |
| Alkyl ketal ester | 0.1-10 % |
| Water | (balance of the formulation) |

The alkyl ketal ester and water can form a cosolvent mixture in this formulation such that the diethylene glycol monobutyl ether (if present in the formulation), and the surfactant are dissolved. The presence of the alkyl ketal ester can allow the amount of diethylene glycol monobutyl ether to be reduced or eliminated.

### General Formulation 4 - Aqueous Toilet Bowl

| | |
|---|---|
| Alkyl ketal ester | 1-10 % |
| Sodium hydroxide | 0 - < 1 %, preferably 0.1 - < 1 |
| Amine oxide type surfactant | 0 - < 5 %, preferably 0.1 - < 5 |
| Sodium hypochlorite | 0 - < 5 %, preferably 0.1 - < 5 |
| Water | (balance of the formulation) |

The alkyl ketal ester is believed to function in this formulation as a cleaning agent and to help solubilize or emulsify other components.

### General Formulation 5 - Aqueous Hard Surface Cleaner

| | |
|---|---|
| Mixture of highly water-miscible and sparingly miscible alkyl ketal esters | 0.1-27 % |
| Sodium xylenesulfonate | 0 - < 2 %, preferably 0.1 - < 2 |
| d-Limonene | 0 - < 2 % |
| Polyethylene glycol (5) undecyl ether | 0 - < 2 %, preferably 0.1 - < 2 |
| C₈₋₁₆ alkyl oxy propaneamine, ethoxylated | 0 - < 2 %, preferably 0.1 - < 2 |
| Water | (balance of the formulation) |

d-Limonene is often used in hard surface cleaners as a degreasing solvent. The alkyl ketal ester can replace or reduce the amount of d-limonene in the formulation. It can also solubilize or emulsify the d-limonene so that higher concentrations of d-limonene can be present, if desired. If called upon to perform a degreasing function in this formulation, the cleaning formulation may contain from 0.1 to 25% of a sparingly soluble alkyl ketal ester (which are generally stronger degreasers than the highly water-miscible types). The highly water- miscible alkyl ketal ester may comprise up to 20% of the formulation, and tends to function as a compatibilizer and/or solublizer for other ingredients, including the sparingly water-miscible alkyl ketal ester.

### General Formulation 6 - Aqueous Glass Cleaner

| | |
|---|---|
| Lower alcohol | 0-7 % |
| Surfactant | 0.5-3% |
| 2-Butoxyethanol | 0-5 % |
| Alkyl ketal ester | 1-10 % |
| Water | (balance of total formulation) |

The alkyl ketal ester and water can form a cosolvent mixture in the formulation into which the lower alcohol and 2-butoxyethanol (if present) are dissolved or dispersed. In some formulations, the alkyl ketal ester can replace both of the lower alcohol and the 2-butoxyethanol.

### General Formulation 7 - General Cleaner Formulation

| | |
|---|---|
| Water | at least 60 % |
| Nonionic surfactant(s) | 0-35, preferably 0.15 - 35 % |
| Anionic surfactant | 0-10, preferably 0.05 - 10 % |
| Alkyl ketal ester | 0.1 - 25 % |
| Water soluble organic solvent | 0 - 20 % |
| Salt | 0.1-5, preferably 0.1 - 2 % |

Highly water-miscible alkyl ketal esters are preferred in this formulation, although sparingly water-miscible alkyl ketal esters can be used, especially if a degreasing function is needed.

### General Formulation 8 - d-Limonene cleaner

| | |
|---|---|
| d-Limonene | 1 - 60 % |
| Surfactant | 10 - 30 % |
| Water | 20 - 65 % |
| Alkyl ketal ester | 0.1 - 10 % |
| Additives | 2 - 12 % |

This cleaning formulation can contain a mixture of a highly water- miscible alkyl ketal ester and a sparingly water-miscible alkyl ketal ester, only the highly water-miscible alkyl ketal ester, or a mixture of both types. The presence of the alkyl ketal esters in this formulation allows one to reduce the d- limonene level (particularly if a sparingly water-miscible alkyl ketal ester is present) and can solubilize or emulsify the d-limonene with the aqueous phase.

### General Formulation 9 - Aqueous Stain Remover

| | |
|---|---|
| Sodium hydroxide | 0.5-1.5 % |
| Acrylic acid polymer / copolymer | 1-5 % |
| Alcohols, C₁₂₋₁₄-secondary, ethoxylated | 0-13 % |
| Alkyl ketal ester | 0.1-25 % |
| Water | 75-90 % |

### General Formulation 10 - Aqueous Foaming Cleaner

| | |
|---|---|
| Chelating agent (e.g., tetrasodium ethylene diaminetetraacetic acid) | 3-7 % |
| Isobutane | 3-7 % |
| Diethylene Glycol Monobutyl Ether | 0-7 % |
| Alkyl ketal ester | 0.1-10 % |
| Anionic surfactant | 1-10 |
| Nonionic surfactant | 0-5 |
| Water | (balance of the formulation) |

### General Formulation 11 - Floor Cleaner

| | |
|---|---|
| Propylene glycol monomethyl ether | 0-15 % |
| Highly water-miscible alkyl ketal ester | 0.1-25 % |
| Water | 55-61 % |
| Oleic acid | 0-17 % |
| Sparingly water-miscible alkyl ketal ester | 0.1-25 % |
| Triethanolamine | 11-13 % |

### General Formulation 12 - Floor Stripper I

| | |
|---|---|
| Propylene glycol monomethyl ether | 0-15 % |
| Highly water-miscible alkyl ketal ester | 0.1-25 % |
| Ammonium hydroxide | 0-10, preferably 1-7 % |
| Anionic surfactant (e.g., sodium sulfate) | ethylhexyl 4-5 % |
| Water | (balance of total formulation) |

The alkyl ketal ester(s) in General Formulations 11 and 12 can perform grease-cutting and other soil removal functions in this formulation and also can allow for the removal or even complete elimination of the propylene glycol monomethyl ether.

### General Formulation 13 - Floor Stripper II

| | |
|---|---|
| Surfactants (anionic/nonionic) | 1-20 % |
| Thickener | 0-5 % |
| Alkali or alkanolamine | 1-40 % |
| Highly water-miscible alkyl ketal ester | 0.1-40 % |
| Water | (balance of total formulation) |

Any of the aforementioned hard surface cleaners, including those of the general formulations 1-13, can be prepared in concentrated form by reducing the amount of water, and correspondingly increasing the concentration of at least the surfactant, and preferably the concentration of both the alkyl ketal ester and the surfactant. Such a concentrated hard surface cleaner formulation may contain up to 60% combined of water and/or volatile organic compound, and more typically contains no more than 40% thereof. Such a concentrated formulation often contains from 5 to 70% or from 10 to 50% by weight of at least one alkyl ketal ester as described herein (preferably including at least one highly water-miscible type) and from 5 to 70% or from 10 to 50% of at least one surfactant.

Hard surfaces of various types can be cleaned using a hard surface cleaner of the invention. The hard surface may be, for example, porcelain or other ceramic material; a metallic surface, a wood surface, a gypsum or other drywall surface, a plastic surface, a concrete or cement surface, or other hard surface. The hard surface may be, for example, a kitchen or bathroom fixture such as a sink, toilet, bath, shower wall, and the like; a hardwood, tile linoleum, vinyl, cement or other floor; an interior wall, a window or mirror, a hard furniture surface, and the like.

An aqueous-based degreaser or oven cleaner is disclosed. Degreasers can be useful for industrial, automotive, marine, aerospace, institutional, office or home use applications.

An aqueous-based degreaser or oven cleaner of the invention includes water, which will in most instances constitute at least 10 % of the weight of the total composition. In some embodiments, water often will constitute at least 30 % by weight of the total composition, and in further embodiments constitute up to as much as 80% by weight of the total composition.

In an aqueous-based degreaser or oven cleaner, an important function of the alkyl ketal ester is that of a solvent for grease or oily soils. Therefore, an aqueous-based degreaser or oven cleaner preferably includes at least one sparingly water-soluble one alkyl ketal ester such as n-propyl-LGK, n-butyl-LGK, ethyl-LPK and ethyl-LEK, as those alkyl ketal esters tend to be more effective degreasers. The sparingly soluble alkyl ketal ester may be present in amounts up to 25 wt. %, and if, present, preferably constitutes from 0.1 to 10 wt. % of the total formulation.

The aqueous-based degreaser or oven cleaner may include a highly water-miscible alkyl ketal ester, preferably one that is miscible in water in all proportions. The highly water-miscible alkyl ketal ester(s) may be present in an amount of up to 40% or more by weight of the total composition of an aqueous-based degreaser and an oven cleaner. In some embodiments, the highly water- miscible alkyl ketal ester selected may not be miscible in water at all proportions; in such a case it preferably is present in the composition in an amount below its miscibility limit. It is preferred that an aqueous degreaser or oven cleaner in accordance with the invention contains at least 1 % of the highly water-miscible alkyl ketal ester thereof, and more preferred that it contain at least 5 weight percent of the highly water-miscible alkyl ketal ester thereof. The highly water- miscible alkyl ketal ester is preferably Me-LGK, MeAcAcGK, Et-AcAcGK or, especially Et-LGK, although mixtures of two or more of the alkyl ketal esters can be used.

An aqueous-based degreaser or oven cleaner may include only water and the alkyl ketal ester(s), possibly in combination with a fragrance and/or a colorant.

An aqueous-based degreaser or oven cleaner formulation may contain, as percentages of the total formulation weight, which is partly outside the claimed range:
a) water: 10-80 %, more typically 30-80 %;
b-1) one or more highly water-miscible alkyl ketal ester(s): 0-40 %, more typically 1-40 %, and more preferably from 5-25 %; the alkyl ketal ester including at least one of Me-LGK, MeAcAcGK, Et-AcAcGK or, Et- LGK;
b-2) one or more sparingly water-miscible alkyl ketal ester(s): 0.1-25 %, more typically 0.1 to 10 %; the alkyl ketal ester preferably including at least one of n-propyl-LGK, n-butyl-LGK, ethyl-LPK and ethyl-LEK;
c) one or more surfactant(s): 0.1-10 %; the surfactants preferably being (1) at least one anionic or nonionic surfactant, (2) one or more nonionic surfactants, (3) a mixture of at least one nonionic surfactant and at least one anionic surfactant, (4) one or more of a Cio-iβ alkyl glycoside, an alkyl betaine or a sulfo succinate salt, or (5) a mixture of (4) with (1), (2) or (3);
e) one or more sparingly water-miscible organic solvent(s); generally from 0-10 %, preferably, if present, 0.1-5 %. This may be, for example, one or more of the alcohols, glycol ethers, hydrocarbons, halogenated hydrocarbons, Cβ-9 alkyl aromatic compounds, olefins, terpenes, terpene oxides, terpenoids, oils, and natural oil derivatives as described above
f) one or more highly water-miscible organic solvents, such as a lower alcohol (e.g., ethanol and 1-propanol), acetone, 0-10 %, preferably, if present, 0.1- 5 %. In some embodiments, the highly miscible organic solvent(s) can often be omitted altogether due to the presence of the alkyl ketal ester(s); and one or more of components d) and g) - p) as described above in regards to the hard surface cleaners. In some embodiments, components d) -p) may be present in any combination of any two or more of them. In some embodiments, any or all of components d) - p) may be omitted in any particular hard surface cleaner formulation.

An exemplary degreaser/oven cleaner formulation follows. The indicated percentages are weight percents based on the total formulation weight. In these formulations, it is preferred that the alkyl ketal ester include one or more sparingly water-miscible alkyl ketal esters, examples of which include methyl-LPK, ethyl-LPK, ethyl LEK, n-propyl-LGK, n-butyl-LGK, and the like. One or more highly water-miscible alkyl ketal esters such as, for example, ethyl-LGK, methyl-LGK, methyl-AcAcGK and ethyl- AcAcGK, may also be present. The sparingly water-miscible alkyl ketal ester(s) in these formulations functions as a degreaser and/or soil solvent. The alkyl ketal esters may also perform other functions compatibilization of ingredients, particularly sparingly water-soluble ingredients into water; formation of a cosolvent mixture in which one or more other ingredients are dissolved or dispersed or elimination or reduction of surfactants and/or organic solvents or others.

### General Formulation 14 - Aqueous Pot and Pan Degreaser

| | |
|---|---|
| Diethylene glycol monobutyl ether | 0-10 % |
| Monoethanolamine (MEA) | 0.1-1 % |
| Carbonate salt | 1-5 % |
| Sparingly water-miscible alkyl ketal ester | 1-10 % |
| Highly water-miscible alkyl ketal ester | 0-25%, preferably 0.1 - 25 % |
| Chelating agent (e.g., sodium dihydrogen citrate) | 1-10 % |
| Anionic surfactants (e.g., sodium cumene sulfonate, alkyl (C₁₀₋₁₆) benzene sulfonic acid, sodium salt | 3-29 % |
| Nonionic surfactant | 0-10% |
| Water | (balance of total formulation) |

The sparingly water-miscible alkyl ketal esters in General Formulation 14 perform as degreasers and/or soil removers and can allow the diethylene glycol monobutyl ether to be reduced or eliminated. The highly water-miscible alkyl ketal ester, if present, can compatibilize other components (including the sparingly soluble alkyl ketal ester) with the water.

A degreaser or oven cleaner of the invention is useful for cleaning any hard substrate which is contaminated with highly hydrophobic materials such as fats, vegetable oils, petroleum products, lubricants and the like, as well as contaminated and degraded products of those types. These surfaces can be, for example, on home, office and/or industrial equipment, in manufacturing and repair facilities (such as automotive repair facilities or other facilities in which petroleum products and/or lubricants are used).

Liquid or gel dishwashing formulations are additional embodiments of the present invention. These dishwashing formulations may be used in hand or machine washing applications. A dishwashing formulation of the invention includes water, which will in most cases constitute at least 50 % by weight of the total composition and may constitute as much as 75 % by weight of the total composition. The liquid or gel dishwashing formulations contain a highly water-miscible alkyl ketal ester, one which is miscible in water at all proportions. The highly water-miscible alkyl ketal ester is Me-LGK, MeAcAcGK, Et-AcAcGK or, especially Et-LGK, although mixtures of two or more of these can be used. Sparingly water-miscible alkyl ketal esters such as n- propyl-LGK, n-butyl-LGK, ethyl-LPK and ethyl-LEK, may be present, in conjunction with one or more highly miscible alkyl ketal esters. Alkyl ketal ester(s) may be present in an amount of up to 25 % or more by weight of a liquid or gel dishwashing formulation.

In some embodiments, the alkyl ketal ester(s) present in the liquid or gel dishwashing formulations may perform any or all of several functions, such as (a) solubilizing or emulsifying the surfactant or other ingredients and (b) functioning as an active cleaning agent.

The liquid or gel dishwashing formulations generally contain at least one surfactant, typically as described with respect to the hard surface cleaner formulations. The surfactant present in hand dishwashing formulations is often an anionic surfactant, or a mixture of one or more anionic surfactants with one or more nonionic surfactants. The surfactant in a machine dishwashing liquid or gel is more typically a nonionic surfactant to reduce foaming. Alternatively (or in addition), the surfactant may include one or more materials derived from plant sources, such as one or more C₁₀₋₁₆ alkyl glycosides, an alkyl betaine, or a sulf o succinate salt.

An aqueous or gel dishwashing formulation may contain, as percentages of the total formulation weight:
a) water: 50-95 %;
b-1) one or more highly water-miscible alkyl ketal ester(s): 1-10 %; the alkyl ketal ester including at least one of Me- LGK, Me-AcAcGK, Et-AcAcGK or, Et-LGK;
b-2) one or more sparingly water-miscible alkyl ketal ester(s): 0-10 %, more typically 0 to 5 %: this alkyl ketal ester preferably including at least one of n-propyl-LGK, n-butyl-LGK, ethyl-LPK and ethyl-LEK, provided that at least one of b-1) and b-2) is present;
c) one or more surfactant(s): 1-10 % but in some cases more preferably from 15- 35 %; the surfactants preferably being (1) at least one anionic surfactant, (2) at least one nonionic surfactant, (3) a mixture of at least one nonionic surfactant and at least one anionic surfactant, (4) one or more of a C₁₀₋₁₆ alkyl glycoside, an alkyl betaine or a sulfo succinate salt, or (5) a mixture of (4) with (1), (2) or (3);
h) one or more builders or chelating agents, particularly a chelating agent such as EDTA or DIDA, 0-30 %, preferably, if present, 0.1-30 %, more preferably, if present, from 1-25 % and still more preferably, if present, from 1-10 %;
i) one or more bleaches, including, for example, a chlorine bleach such as sodium hypochlorite or a non-chlorine bleach: 0-10 %, preferably, if present 0.1-5 %;
o) one or more abrasives, 0-30 %, preferably if present 1-20 %.

In addition, an aqueous or gel dishwashing formulation may contain any of components d) - g), j)-n) or p) as described before with respect to the hard surface cleaners, singly or in any combination of any two or more of them. Any or all of components d) - g), j) - n) and p) may be omitted in any particular dishwashing formulation.

Some exemplary dishwashing formulations follow. The indicated percentages are weight percents based on the total formulation weight. In these dishwashing formulations, the alkyl ketal ester include at least one highly water-miscible alkyl ester, including ethyl-LGK, methyl-LGK, methyl-AcAcGK and ethyl-AcAcGK. Sparingly water-miscible alkyl ketal esters such as, for example, methyl-LPK, ethyl-LPK, ethyl LEK, n-propyl-LGK, n-butyl-LGK and the like, may be present, especially for their grease-cutting function. These dishwashing formulations may contain one or more highly water-miscible alkyl ketal esters together with one or more sparingly miscible alkyl ketal esters. As before, the alkyl ketal esters may perform various functions in these dishwashing formulations, in addition to grease cutting. These include one or more of (1) soil dissolution and/or removal; (2) compatibilization of ingredients, particularly sparingly water-soluble ingredients into water; (3) formation of a cosolvent mixture in which one or more other ingredients are dissolved or dispersed, (4) elimination or reduction of surfactants and/or organic solvents or others.

### General Formulation 15 - Aqueous Gel Dishwasher

| | |
|---|---|
| Base (sodium hydroxide) | < 1.0 % |
| Alkyl ketal ester | 0.1 to 25 %, preferably 1 - 10 % |
| Anionic surfactant (e.g., alkyl benzenesulfonate) | 0.1 to < 25 % |
| Thickening agent (xanthan gum) | 1-3 % |
| Abrasive | 0-30 % |
| Water | (balance of the formulation) |

### General Formulation 16 - Aqueous Liquid Hand Dish Detergent

| | |
|---|---|
| Alkyl ketal ester | 0.1-25 % |
| Lower alcohol | 0-10 % |
| Sodium sulfate | 0-15, preferably 0.1-2 % |
| Anionic surfactant (e.g., sodium dodecyl benzenesulfonate) | 1-30%, preferably 1-20 % |
| Nonionic surfactant | 0-25%, preferably 1-15% |
| Water | (balance of the formulation) |

### General Formulation 17 - Aqueous Liquid Machine Dish Detergent II

| | |
|---|---|
| Alkyl ketal ester | 0.1-5 % |
| Nonionic surfactant | 1-40% |
| Sodium silicate | 1-30% |
| Sodium carbonate | 1-25% |
| Hydrogen Peroxide | 0-5% |
| Water | (balance of the formulation) |

### General Formulation 18 - Dish Detergent Microemulsion

| | |
|---|---|
| Water | 10-40 % |
| Surfactant | 1-20 % |
| Secondary or tertiary C₄-C₅ alcohol | 0-50 % |
| Highly water-miscible alkyl ketal ester | 0.1-25 % |
| Terpene | 0-88 % |
| Sparingly water-miscible alkyl ketal ester | 0.1-50 % |

The alkyl ketal esters in General Formulations 15-18 can perform as degreasers and/or soil removers, especially if one of them is a sparingly water- soluble type. The alkyl ketal ester also can compatibilize other components (including the sparingly soluble alkyl ketal ester) with the water, and can allow the other solvents (alcohols, terpenes and the like) to be reduced or eliminated.

In other embodiments of the present invention, the cleaning composition is a liquid laundry product, which may be for hand or machine washing. A liquid laundry formulation of the invention includes water, which will in most cases constitute at least 60 % by weight of the total composition. Water often will constitute at least 75 % by weight of the total composition and constitute as much as 95 % by weight of the total composition. Liquid laundry formulations contain a highly water-miscible alkyl ketal ester, Me-LGK, Me-AcAcGK, Et-AcAcGK or, especially Et-LGK, although mixtures of two or more of these can be used. The highly miscible alkyl ketal ester(s) may be present in an amount of up to 10 % by weight of the liquid laundry product. If the highly miscible alkyl ketal ester is not miscible in water at all proportions, it preferably is present in an amount below its miscibility limit. In some embodiments, the liquid laundry product formulation includes one or more of the highly miscible alkyl ketal esters present in conjunction with a sparingly miscible alkyl ketal ester, such as n-propyl-LGK, n-butyl-LGK, ethyl- LPK and ethyl-LEK. It is preferred that the composition contains at least 1 % of alkyl ketal ester(s), and more preferred that it contain at least 5 weight percent thereof.

In liquid laundry products, the alkyl ketal ester(s) may perform any or all of several functions, such as solubilizing or emulsifying the surfactant or other ingredients and functioning as an active cleaning agent. A liquid laundry product contains at least one surfactant, typically as described with respect to the hard surface cleaners above. Builders and/or proteolytic enzymes are commonly present in liquid laundry products, as are chlorine and non-chlorine bleaches.

A liquid laundry formulation may contain, as percentages of the total formulation weight:
a) water: 50-95 %;
b-1) one or more highly water-miscible alkyl ketal ester(s): 0.1-10 %, more typically 1-10 %; the alkyl ketal ester preferably including at least one of Me- LGK, Me-AcAcGK, Et-AcAcGK or, Et-LGK;
b-2) one or more sparingly water-miscible alkyl ketal ester(s): 0-10 %, more typically 0 to 5 %: this alkyl ketal ester preferably including at least one of n-propyl-LGK, n-butyl-LGK, ethyl-LPK and ethyl-LEK;
c) one or more surfactant(s): 1-10 %, but in some cases more preferably from 15- 35 %; the surfactants preferably being (1) at least one anionic surfactant, (2) a nonionic surfactant, (3) a mixture of at least one nonionic surfactant and at least one anionic surfactant, (4) one or more of a C₁₀₋₁₆ alkyl glycoside, an alkyl betaine or a sulfo succinate salt, or (5) a mixture of (4) with (1), (2) or (3);
h) one or more builders or chelating agents, particularly a chelating agent such as EDTA or DIDA: 0-30 %, preferably, if present, 0.1-30 %, more preferably, if present, from 1-25 % and still more preferably, if present, from 1-10 %;
i) one or more bleaches, including, for example, a chlorine bleach such as sodium hypochlorite or a non-chlorine bleach:0-10 %, preferably, if present 0.1-5%;
o) one or more abrasives: 0-30 % preferably if present 1-20 %
p) one or more anti-redeposition additives, such as, for example carboxymethylcellulose salts and cellulose acetate polymeric agents, 0-5%, preferably if present 0.1 to 2%; and
q) brightening agents, including optical brightening agents, fluorescent brightening agents and fluorescent whitening agents, including, for example, sulfonated triazine-stilbenes, coumarins, imidazolines, diazoles, triazoles, benzoxazolines and biphenyl stilbenes, 0-3, preferably if present 0.1 to 1.

In addition, a liquid laundry product may contain any of components e) - g), j)-n) or p) as listed before, singly or in any combination of any two or more of them. Any or all of components e)-g), j) - n) and p) may be omitted in any particular liquid laundry formulation. In particular, an advantage of this invention is that component(s) e) and f) can be omitted from the product; their functions being taken over the by the alkyl ketal esters present in the formulation.

Some exemplary liquid laundry formulations follow. The indicated percentages are weight percents based on the total formulation weight. In these liquid laundry formulations, the alkyl ketal ester include at least one highly water-miscible alkyl ester, including including ethyl-LGK, methyl-LGK, methyl-AcAcGK and ethyl- AcAcGK. These dishwashing formulations may contain one or more highly water- miscible alkyl ketal esters together with one or more sparingly miscible alkyl ketal esters. The function of these alkyl ketal esters in liquid laundry formulations is similar to those described before with respect to hard surface cleaners.

### General Formulation 19 - Aqueous Liquid Laundry Detergent

| | |
|---|---|
| Sodium hydroxide | 0-1 % |
| Sodium chloride | ∼ 1 % |
| Nonionic surfactant (e.g., ethoxylated alcohol (C₁₂₋₁₆)) | 0-20, preferably 0.1-6 % |
| Anionic surfactant (e.g., benzenesulfonic acid, C₁₀₋₁₆ alkyl) | 1-20, preferably 1-10 % |
| Builder(s) | 0-10 |
| Anti-redeposition agent | 0-1% |
| Proteolytic enzymes | 0-2 |
| Brightener (fluorescing agent) | 0-1 |
| Buffer(s) | 0-5 |
| Alkyl ketal ester | 0.1-25 % |
| Water | (balance of total formulation) |

### General Formulation 20 - Aqueous Laundry Stain Remover

| | |
|---|---|
| Proteolytic enzyme | 0 - < 1 % |
| Surfactant | 10-20 % |
| Chelating agent | 0.5-1.5 % |
| Alkyl ketal ester | 1-10 % |
| Water | (balance of total formulation) |

### General Formulation 21 - Aqueous Dry-cleaning Composition

| | |
|---|---|
| Water | 60-95 % |
| Polyacrylates | 0.2-0.5 % |
| Mixed glycol ethers | 0-30 % |
| Alkyl ketal ester | 0.1-25 % |
| Surfactant(s) | > 0.1 % |
| 1,2-octanediol | 0.01-5 % |

Any of the aforementioned liquid laundry formulations, including those of the general formulations 19 to 21, can be prepared in concentrated form by reducing the amount of water, and correspondingly increasing the concentration of at least the surfactant, and preferably the concentration of both the alkyl ketal ester and the surfactant. Such a concentrated hard surface cleaner formulation may contain up to 50% combined of water and/or volatile organic compound, and more typically contains no more than 40% thereof. Such a concentrated formulation often contains from 5 to 70% or from 10 to 50% by weight of at least one alkyl ketal ester as described herein (preferably including at least one highly water-miscible type) and from 5 to 70% or from 10 to 50% of at least one surfactant.

In other embodiments the cleaning compositions are carpet or other soft surface cleaning products. These products may contain:
a) water: 10-90 %;
b-1) one or more highly miscible alkyl ketal ester(s): 0.1-10 %, more typically 1-10 %; the alkyl ketal ester including at least one of Me- LGK, MeAcAcGK, Et-AcAcGK or, Et-LGK;
b-2) one or more sparingly miscible alkyl ketal ester(s): 0-25 %, more typically if present, 0.1 to 10 %: this alkyl ketal ester preferably including at least one of n-propyl-LGK, n-butyl-LGK, ethyl-LPK and ethyl-LEK;
c) one or more surfactant(s): 0.1-10 %; the surfactants preferably being (1) at least one anionic surfactant, (2) a mixture of at least one nonionic surfactant and at least one anionic surfactant, (3) one or more of a C₁₀₋₁₆ alkyl glycoside, an alkyl betaine or a sulfo succinate salt, or (4) a mixture of (3) with (1) or (2). These formulations also often contain at least one builder, which may constitute as much as 50 % of the weight of the formulation, and may contain any one or more of components d) - p) as described before. Any of those components d) - p) may be absent from the product. Once again, an advantage of this invention is that components e) and f) may be eliminated from the formulation.

Some exemplary carpet or soft surface cleaning formulations follow. The indicated percentages are weight percents based on the total formulation weight. In these formulations, the alkyl ketal ester includes at least one highly water-miscible alkyl ester, including, ethyl-LGK, methyl-LGK, methyl-AcAcGK and ethyl-AcAcGK. However, a sparingly water-miscible alkyl ketal ester can be used instead (or in addition) if stronger cleaning ability is needed. The function of these alkyl ketal esters in carpet and soft surface formulations is similar to those described before with respect to hard surface cleaners.

### General Formulation 22 - Aqueous Carpet Cleaner

| | |
|---|---|
| Water | 68 - 89 % |
| Builder | 1 - 3 % |
| Anionic surfactant | 6 - 8 % |
| Glycol ether PnB | 0 - 6 % |
| Alkyl ketal ester | 0.1 - 25 % |

In this formulation, the alkyl ketal ester can allow the amount of glycol ether PnB to be reduced or eliminated.

### General Formulation 23 - Carpet Cleaner

| | |
|---|---|
| Detergent builder | 0.1-50 % |
| Highly water-miscible alkyl ketal ester | 0.1-25 % |
| Fatty acid alkyl ester / dibasic ester | 0-10 % |
| Sparingly water-miscible alkyl ketal ester | 0.1-25 % |
| Surfactant | 0.1-10% |
| Water | (balance of total formulation) |

### General Formulation 24 - Carpet Shampoo

| | |
|---|---|
| Water | 58-80 % |
| Anionic Surfactant | 6-8 % |
| Emulsifier/buffer | 9-12 % |
| Glycol ether PM | 0-5 % |
| Highly water-miscible alkyl ketal ester | 0.1-25 % |
| Potassium oleate | 2-5 % |

### General Formulation 25 - Carpet Shampoo-Medium Foam Cleaner

| | |
|---|---|
| Water | 68-89 % |
| Glycol ether DPM | 0-6 % |
| Highly water-miscible alkyl ketal ester | 0.1-25 % |
| Builder (trisodium phosphate) | 1-3 % |
| Sodium dodecyl sulfate (surfactant) | 6-8 % |

In this formulation, the alkyl ketal ester can allow the amount of glycol ether DMB to be reduced or eliminated.

### General Formulation 26 - Leather, Vinyl, Plastic Cleaner

| | |
|---|---|
| Water | 77-84 % |
| Highly water-miscible alkyl ketal ester | 0.1-25 % |
| Ethylene glycol monobutyl ether | 0-6 % |
| Propylene glycol monomethyl ether | 0-15 % |
| Sparingly water-miscible alkyl ketal ester | 0.1-25 % |
| Isopropanol | 0-3 % |
| Polyethylene glycol | 0-17 % |
| Surfactant | 0-10, preferably 0.1-5 |
| Amyl acetate | 0 - 2 % |

In this formulation, the alkyl ketal ester can allow the amount of isopropanol, amyl acetate and the glycol ethers to be reduced or eliminated, thereby greatly simplifying the formulation.

Any of the aforementioned carpet or soft surface cleaners, including those of the general formulations 22-26, can be prepared in concentrated form by reducing the amount of water, and correspondingly increasing the concentration at least the surfactant, and preferably the concentration of both the alkyl ketal ester and the surfactant. Such a concentrated carpet or soft surface cleaner formulation may contain up to 60% combined of water and/or volatile organic compound, and more typically contains no more than 40% thereof. Such a concentrated formulation often contains from 5 to 70% or from 10 to 50% by weight of at least one alkyl ketal ester as described herein (preferably including at least one highly water-miscible type) and from 5 to 70% or from 10 to 50% of at least one surfactant.

Carpet and soft- surface cleaners are useful for cleaning various textile products, including carpets, rugs, other textile floor coverings, curtains, fabric wall coverings, window shades, upholstery, leather and artificial leather products, and the like.

Outside the present invention is a powdered cleaning product such as a powdered laundry detergent, a powdered or granulated dishwashing product, a powdered bleaching cleanser, and the like.

These products are generally characterized as containing at least one surfactant, an alkyl ketal ester which may be highly miscible or sparingly miscible in the formulation, and a solid phase which typically includes one or more of a carrier, an abrasive, a detergency builder or a solid bleach component. Powdered cleaning products typically contain from 1 to 40 %, preferably from 5 to 30 %, more preferably from 10 to 25%, of a surfactant. They typically contain from 1 to 40 %, preferably from 5 to 30 % and more preferably from 10 to 30 % of a builder. These products typically contain less than 5 % by weight water and usually contain no more than about 2 % by weight water. These cleaning products also often contain chlorine or a non-chlorine bleach and/or a proteolytic enzyme. Other components as described with respect to the hard surface cleaners may also be present, consistent with the solid powdered form.

Some exemplary powdered cleaning formulations follow. The indicated percentages are weight percents based on the total formulation weight. In these formulations, it is preferred in most cases that the alkyl ketal ester includes at least one highly water-miscible alkyl ester, such as, for example, ethyl-LGK, methyl-LGK, methyl-AcAcGK and ethyl-AcAcGK. However, a sparingly water-miscible alkyl ketal ester can be used instead (or in addition) if stronger cleaning ability is needed. The function of these alkyl ketal esters in powdered formulations is similar to those described before with respect to hard surface cleaners.

### General Formulation 27 - Dishwasher Powder

| | |
|---|---|
| Alkyl ketal ester | 0.1-25 % |
| Nonionic surfactant(s) | 4-8 % |
| Chelating agent (e.g., citric acid) | 2-4 % |
| Sodium carbonate | 0.1 - < 25 % |
| Sodium perborate | 0-14 % |
| Pentasodium tripolyphosphate | 0.1 - < 35 % |
| Proteolytic enzyme | 0 - < 2 % |
| Anionic surfactant(s) | 0-10, preferably 2-10 % |
| Sodium silicate | > 25 % |
| Sodium sulfate anhydrous | 1-15 % |
| Colorant/Pigment/Dye | 0-2 % |
| Bleach (including sodium hypochlorite) | 0.1-10 % |
| Potassium hydroxide | 0-5 % |

### General Formulation 28 - Bleaching Powdered Cleaner

| | |
|---|---|
| Calcium carbonate | > 60 % |
| Quartz | 0.1-1 % |
| Surfactant | < 25 % |
| Alkyl ketal ester | 1-10 % |
| Bleach (organic Cl_{2/}O₂) | 1-10 % |

### General Formulation 29 - Laundry Powder

| | |
|---|---|
| Alkyl ketal ester | 1-10 % |
| Nonionic surfactant | 3-7 % |
| Alkali metal carbonate | 15-40 % |
| Proteolytic enzyme | 0.1 % |
| Anionic surfactant | 7-13 % |
| Abrasive (e.g., zeolites) | 20-40% |
| Sodium metasilicate | 20-40% |

In other embodiment of the invention, the cleansing composition is a disinfectant, decontaminating agent and/or antistatic agent. Some exemplary cleaning formulations of these types follow. The indicated percentages are weight percents based on the total formulation weight. In these formulations, the alkyl ketal ester includes at least one highly water-miscible alkyl ester, including ethyl-LGK, methyl-LGK, methyl-AcAcGK and ethyl-AcAcGK. However, a sparingly water-miscible alkyl ketal ester can be used instead (or in addition) if stronger cleaning ability is needed. The function of these alkyl ketal esters in powdered formulations is similar to those described before with respect to hard surface cleaners.

### General Formulation 30 - Aqueous Quaternary Amine Sanitizer

| | |
|---|---|
| Ethanol/SD Alcohol 40 | 0-5 % |
| Antimicrobials (e.g., dodecyl dimethyl ammonium chloride, alkyl (C₁₂₋₁₆) dimethylbenzylammonium chloride) | 1-15 % |
| Alkyl ketal ester | 1-10 % |
| Nonionic or amphoteric surfactant | 0-10%, preferably 0.1-5% |
| Water | (balance of total formulation) |

### General Formulation 31 - Antistatic Dusting Spray

| | |
|---|---|
| 1-propoxy-2-propanol | 0-4 % |
| Alkyl ketal ester | 0.1-25 % |
| Preservative(s) | < 1 % |
| Cyclodextrin | < 1 % |
| Water | (balance of total formulation) |

A cleaning composition of the present invention may be incorporated onto an absorbent material for purposes of easy application and/or dosing. The choice of absorbent material may vary broadly depending on the intended use. Thus, for cleansing the skin, the cleansing and disinfecting composition may be incorporated into a cotton pad, a fabric pad or a sponge. For cleansing surfaces other than the skin, it is advantageous to incorporate the cleansing and disinfecting composition into a sponge, foam or a nonwoven fabric. A sponge may have an abrasive surface. An example of a cleaning formulation for application to a wipe or sponge follows. The indicated percentages are weight percents based on the total formulation weight. The alkyl ketal ester for application to a wipe or sponge includes at least one highly water-miscible alkyl ester, including ethyl-LGK, methyl-LGK, methyl-AcAcGK and ethyl- AcAcGK. However, a sparingly water-miscible alkyl ketal ester can be used in addition if stronger cleaning ability is needed. The function of these alkyl ketal esters in powdered formulations is similar to those described before with respect to hard surface cleaners.

### General Formulation 32 - General Cleaner/Moistened Wipes Composition

| | |
|---|---|
| 2-Butoxyethanol | 0-5 |
| Antimicrobial (e.g., alkyl (C₁₂₋₁₆) dimethyl benzylammonium chloride | 0.1-0.3 |
| Nonionic or amphoteric surfactant | 0-10, preferably 0.1-5 |
| Alkyl ketal ester | 0.1-10 |
| Water | (balance of formulation) |

The alkyl ketal esters can in this formulation permit the 2-butoxyethanol to be reduced or eliminated from this formulation.

In other specific embodiments, the cleaner of the invention is a personal care cleaning product for cleaning human skin, hair or other tissues. Examples of these include face and/or hand cleansers, body washes, feminine hygiene products, makeup removers and various types of shampoos, including baby shampoos, dandruff shampoos, conditioning shampoos and the like. A personal care cleaning product according to the invention include water, which will constitute at least 50 % by weight of the total composition.

A personal care cleaning product typically contains one or more emollients, by which is meant a material that softens the skin. Among the emollients are materials within one or more of classes s)- w), as follow:
s) paraffinic, naphthenic or aromatic mineral oil;
t) nonionic organic compounds which have a melting temperature of less than 45°C, has a molecular weight of at least 190, contains at least one amido, or ester group and at least one alkyl chain containing at least 8 carbon atoms, and has a solubility in water of no greater than 1 part in 99 parts of water. These include a range of natural oils and synthetic ester and amide products which have little or no surfactant properties (having an HLB of less than 4, preferably less than 2) and which often function as emollients in a personal care cleaning product.

Examples of materials t) include vegetable oils and animal fats and derivatives thereof, C₈₋₂₄ linear or branched alkyl esters of C-₋₂₄fatty acids, di-C₈₋₂₄ esters of dicarboxylic acids, C₈₋₂₄ fatty acid esters of C₈₋₂₄ linear or branched alkanoic acids, C₈₋₂₄, especially C₁₂₋₁₅ alkyl benzoates, poly(propylene oxide) esters of C₈₋₂₄ fatty acids, di-C₈₋₂₄ linear or branched alkyl esters of aromatic diacids, di- C₈₋₂₄ fatty acid esters of aromatic diacids, and the like; C₈₋₂₄ linear or branched alkyl amides of C₈₋₂₄ fatty acids, di-C₈₋₂₄ amides of dicarboxylic acids, C₈₋₂₄ fatty acid amides of C₈₋₂₄ linear or branched alkanoic acids, poly(propylene oxide) amides of C₈₋₂₄ fatty acids, di-C₈₋₂₄ linear or branched alkyl amides of aromatic diacids, di-C₈₋₂₄ fatty acid amides of aromatic diacids, and the like;
u) nonionic organosilicone compounds which have a melting temperature of less than 45°C and has a solubility in water of no greater than 1 part in 99 parts of water. Examples are dimethicone and cyclopentasiloxane;
v) long chain (eight or more carbon atoms) alcohols such as 1-octanol, 1- decanol, 1-docecanol, cetyl alcohol and the like. These alcohols can perform various functions, including thickening and a solvating/compatibilizing function, and can function as emollients as well;
w) waxes. Waxes include synthetic or mineral waxes such as ceresin, montan, ozocerite, peat, paraffin, microcrystalline, polypropylene and other polymerized poly-α-olefin waxes, substituted amide, petroleum jelly, esterified or saponified waxes, and the like; and waxes of plant or animal origin.

When present, components s) through w) may, in the aggregate, constitute from 0.5 to 50% by weight of a personal care cleaning product. Materials o) through s) tend to be highly hydrophobic, and so products containing these materials and water tend to be in the form of emulsions.

A personal care cleaning product contain one or more surfactants, especially for general-purpose skin cleaners and shampoo products. Makeup removers in accordance with the invention may be devoid of a surfactant in some embodiments. Any of the surfactants described before can be present in a personal care cleaning product, including the various sulfate and sulfonate surfactants, the sulf o succinate surfactants, the betaine derivatives, and the like. The personal care product typically contains at least one "primary" surfactant that promotes foaming and cleansing, such as isothianate, sulfate or sulfonate surfactants. A "secondary" or "foam boosting" surfactant such as a betaine, sarcosinate, sulf o succinate, taurate, ether sulfate, glucoside, amine oxide, sultaine, lactylate and/or glutamate surfactant may be present, often in conjunction with a "primary surfactant".

A personal care cleaning product of the invention includes at least one highly water-miscible alkyl ketal esters including Me-LGK, MeAcAcGK, Et- AcAcGK or, especially Et-LGK, although mixtures of two or more of alkyl ketal esters can be used. In some embodiments, the highly water-miscible alkyl ketal esters may be present in conjunction with a sparingly soluble alkyl ketal ester such as, for example, n-propyl-LGK, n-butyl-LGK, ethyl-LPK and ethyl-LEK. The amount of alkyl ketal ester present in a personal care cleaning product is from 1 % to 10 % by weight of the total composition, more preferably from about 3 % to 10 % by weight of the total composition, or from about 2 % to 10 % by weight of the total composition.

The function of the alkyl ketal ester in a personal care cleaning product may be that of an emulsifier and/or cosolvent, which increases the stability of the product and/or solubilizes one or more materials into another. Highly water- miscible alkyl ketal esters in particular perform this emulsifying and/or cosolvent function very well. Sparingly water-miscible alkyl ketal esters can perform a cosolvent function, a viscosity reducing function, and often are every effective cleaning agents. Personal care cleaning products that contain at least 1% by weight of sparingly soluble alkyl ketal esters are very effective makeup removers. Components d)-w) as described above may be present in a personal care cleaning product of the invention. Any two or more of them can be present in combination. Any or all of components d) - w) may be omitted in any particular personal care cleaning product formulation. Component d) is typically present in a spray formulation. It is noted earlier that in some instances, a single material may perform multiple functions in a personal care cleaner product.

In addition, a personal care cleaning product may contain one or more hair conditioners, such as various polyquat compounds and silicone compounds like silicone copolyols and amino functional silicones; one or more opacifiers such as a glycol stearate or a glycol distearate, one or more specific functional actives such as anti-dandruff, anti-acne, skin whiteners, vitamins, aloe, antioxidants, UV absorbers and other actives, one or more thickeners or suspending agents, and the like.

Some exemplary personal care cleaner formulations follow. The indicated percentages are weight percents based on the total formulation weight.

### General Formulation 33 - Mild Skin Cleanser

| | | |
|---|---|---|
| Part A: | Warm distilled Water | 40 - 75 % |
| Part B: | Sulfosuccinate | 4 - 30 % |
| | Cocobetaine | 4 - 30 % |
| | PEG-7 Glyceryl Cocoate | 1 - 10 % |
| | PEG-150 Distearate | 0.5 - 5 % |
| Part C: | Phenoxyethanol | 0.1 - 2 % |
| Highly water-miscible alkyl ketal ester | | 0.1 - 20% |
| Sparingly water-miscible alkyl ketal ester | | 0 - 10% |

### General Formulation 34 - Aloe Vera Face Cleansing Lotion

| | | |
|---|---|---|
| Part A: | Warm Distilled Water | 45 - 75 % |
| Part B: | Sulfosuccinate | 4 - 30 % |
| | Cocobetaine | 4 - 30 % |
| | Glycol Stearate IP | 2 - 6 % |
| Part C: | Aloe Vera 10xConcentrate | 0.1 - 5 % |
| | Phenoxyethanol/SA | 0.1 - 2 % |
| Highly water-miscible alkyl ketal ester | | 0.1 - 20% |
| Sparingly water-miscible alkyl ketal ester | | 0 - 10% |

The alkyl ketal ester(s) can be added into any of all of parts A, B and C of General Formulation 33 or 34. The highly water-miscible alkyl ketal ester can function as a cosolvent with the water to solubilize or compatibilize other ingredients, notably the part B materials and the phenoxyethanol. In some case, the presence of the highly water-miscible alkyl ketal ester can allow the concentration of the part B materials and the phenoxyethanol to be increased to form a more highly concentrated product.

### General Formulation 35 -Shampoo

**Part A:**

| | |
|---|---|
| Ammonium or Sodium Lauryl sulfate | 20 - 50 % |
| Ammonium or Sodium laureth Sulfate | 15 - 30 % |
| Sodium lauroyl sarcosinate | 2 - 10 % |
| Cocamidopropylamine oxide | 2 - 10 % |
| PPG ester or PEG ester | 0.1 - 3 % |
| Polyquat aqueous solution | 1 - 3 % |
| Ethylene glycol distearate | 1 - 3 % |

**Part B:**

| | |
|---|---|
| Botanical extract/derivative solution & carrier | 0 - 1 % |
| Hydrolyzed silk solution and carriers | 0.05 - 0.2 % |
| Hydrolyzed Keratin or Silk Protein | 0 - 2 % |
| Methylisothiazolinone | 0 - 0.2 % |

**Part C:**

| | |
|---|---|
| Cocamidopropy Hydroxylsultaine | 5 - 15 % |
| Cocamidopropyl Betaine | 3 - 10 % |
| Highly water-miscible alkyl ketal ester | 0.1 - 20% |
| Sparingly water-miscible alkyl ketal ester | 0 - 10% |

### General Formulation 36 - Baby Shampoo

**Part A:**

| | |
|---|---|
| Ethoxylated Glycerides | 10 - 20 % |
| Ethoxylated Thickener | 0 - 2 % |

**Part B:**

| | |
|---|---|
| Sodium Laureth Sulfate | 10 - 30 % |
| Cocamidopropyl Hydroxysultaine | 8 - 15 % |
| Deionized Water | 40 - 75 % |

**Part C:**

| | |
|---|---|
| Propylene Glycol & Dizolidinyl Urea & Methyl Paraben & Paraben | 0.1 - 2 % |
| HCl, 10% solution | q.s. |
| Highly water-miscible alkyl ketal ester | 0.1 - 20% |
| Sparingly water-miscible alkyl ketal ester | 0 - 10% |

The alkyl ketal ester(s) can be added into any of all of parts A, B and C of General Formulation 35 or 36. The highly water-miscible alkyl ketal ester can function as a cosolvent with the water to solubilize or compatibilize other ingredients, notably the part B materials and the phenoxyethanol. In some case, the presence of the highly water-miscible alkyl ketal ester can allow the concentration of the part B materials to be increased, to form a more concentrated shampoo product.

Any of formulations 1-36 above may contain aesthetic agents such as colorants and/or fragrances.

Cleaning products in accordance with the invention can be used in any convenient manner. Cleaning is generally accomplished by bringing the cleaning product into contact with a soiled substrate and then removing the cleaning product together with removed soils. The cleaning product may be diluted with water or other solvent before being applied, especially if the cleaning product is in concentrated form. Removing can be accomplished, for example, by one or more steps of wiping, vacuuming, mechanical removal (as via a cloth, mop broom or sponge, for example), absorption onto an absorbent, and/or rinsing. If necessary, mechanical soil removal steps such as spraying, rubbing, brushing and/or scrubbing can be performed in order to further loosen and pick up soils from the substrate.

The present invention is more particularly described in the following examples that are intended as illustrations only, since numerous modifications and variations within the scope of the present invention will be apparent to those skilled in the art. Unless otherwise noted, all parts, percentages, and ratios reported in the following examples are on a weight, and all reagents used in the examples were obtained, or are available, from chemical suppliers described below, or may be synthesized by conventional techniques. In the following examples, ethyl-LGK or Et-LGK corresponds to the alkyl ketal ester formed from ethyl levulinate and glycerine; ethyl-LPK or Et-LPK refers to the alkyl ketal ester formed from ethyl levulinate and 1,2-propylene glycol.

### Example 1 - Bleach Stability Evaluations with Et-LPK

The purity of an Et-LPK sample is measured by GC-FID (Model No. 7890A Gas Chromatograph w/ Flame Ionization Detector; Agilent Technologies, Santa Clara, California). In a 20 ml vial (borosilicate scintillation; VWR International, West Chester, Pennsylvania), 2 ml of a 6% sodium hypochlorite (bleach; Clorox Co., Oakland, California) in water is mixed with 8 ml of Et-LPK to form a mixture. The mixture is shaken vigorously to produce a nearly clear solution. The mixture is further stirred overnight on a magnetic stir plate with a magnetic stir bar. Upon standing, the mixture separates into two phases - an upper organic phase and a bottom aqueous phase. A sample of the organic phase is analyzed by GC-FID to determine the purity of the Et-LPK after mixing. GC- FID results showed little to no change in the purity of Et-LPK when compared to the initial Et-LPK purity, indicating that Et-LPK is stable in solutions having concentrations of sodium hypochlorite commonly found in consumer bleach products.

### Example 2-3 - Bleach and Peroxide Stability Evaluations with Et-LGK

Examples 2-3 and a control (non-bleach) formulation are prepared by combining the ingredients indicated in Table 1 in a 20 ml borosilicate glass scintillation vial, followed by shaking vigorously to form a clear, colorless solution. All formulations are clear, colorless homogenous solutions after standing for three days at room temperature.

**Table 1**

| **Control (non-bleach)** | **Amount (g)** |
|---|---|
| Water, DI | 9.01 |
| Sodium citrate dehydrate | 0.10 |
| Et-LGK | 1.01 |
| | |

| **Example 1** | **Amount (g)** |
|---|---|
| Water, DI | 7.03 |
| Sodium citrate dehydrate | 0.10 |
| Et-LGK | 1.00 |
| 6% sodium hypochlorite solution in water | 1.99 |
| | |

| **Example 2** | **Amount (g)** |
|---|---|
| Water, DI | 8.81 |
| Sodium citrate dehydrate | 0.10 |
| Et-LGK | 1.00 |
| Hydrogen peroxide, 50% solution in water | 0.22 |

After storing for 96 hours, GC/FID analysis is performed to assess the stability of Examples 2 and 3 and Comparative Sample A. Three analyses are performed with each test sample. For the control, it is found that the concentration of Et-LGK is about 10%. For Example 1, the Et-LGK concentration is about 9.4%, indicating a slight degradation of the material in the presence of the chlorine bleach. For Example 2, the Et-LGK concentration is the same as the control, indicating no interaction with the hydrogen peroxide bleach.

### Example 4 -Et-LPK Solubilization

Several three-component mixtures of Et-LGK, water and Et-LPK are prepared by mixing the materials the above materials at room temperature in a 20 ml scintillation vial with shaking. The mixtures prepared are analyzed visually for miscibility. Clear mixture are considered to be "miscible", and mixtures, which are cloudy or form two or more distinct phases are considered to be "immiscible" on this test. Results for the various mixtures are described in Table 2.

**Table 2**

| **Mixture No.** | **Result** | **% Et-LGK** | **% Et-LPK** | **% Water** |
|---|---|---|---|---|
| 11 | Cloudy/Immiscible | 0.0 | 10.9 | 89.1 |
| 12 | Miscible | 50.0 | 5.0 | 45.0 |
| 13 | Miscible | 67.8 | 10.1 | 22.1 |
| 14 | Miscible | 59.8 | 10.7 | 29.5 |
| 15 | Miscible | 59.2 | 20.4 | 20.4 |
| 16 | Miscible | 59.2 | 29.9 | 10.9 |
| 17 | Miscible | 53.5 | 35.7 | 10.8 |
| 18 | Miscible | 40.0 | 55.0 | 5.0 |

As shown in Table 2, Et-LPK is immiscible in water at the 10.9% level. A mixture of water and Et-LGK mixture is a much better solvent for Et-LPK than is water alone. Et-LPK concentrations of nearly 55 wt. % can be obtained in a miscible mixture, as shown by Mixture 18.

### Example 5 and Comparative Samples A-C - Isobutyl Isobutyrate (IBIB) Solubilization

Mixtures of water and isobutyl isobutyrate (IBIB; Acros Organics, a division of Thermo Fisher Scientific; Waltham, Massachusetts) are prepared at 10/90, 25/75, 50/50, 75/25 and 90/10 volume ratios. All form immiscible mixtures. To assess the ability of Et-LGK to solubilize IBIB into water, Et-LGK is added to each of these mixtures with vigorous stirring in a 20 mL scintillation vial at about 25°C until a clear solution is obtained. The Et-LGK concentration (in volume %) needed in each case to form a clear solution is reported in Table 3.

For comparison, the abilities of propylene glycol methyl ether (Acros Organics, a division of Thermo Fisher Scientific; Waltham, Massachusetts), ethylene glycol monobutyl ether (Sigma -Al drich; St. Louis, Missouri) and dipropylene glycol methyl ether (Sigma-Aldrich; St. Louis, Missouri) to form a clear solution with water and IBIB are evaluated the same way. The concentrations of these comparative solvents (in volume-%) needed in each case to form a clear solution is reported in Table 3 as Comparative Samples A, B and C, respectively.

**Table 3**

| | Example or Comparative Sample | | | |
|---|---|---|---|---|
| | 5 | A* | B* | C* |
| Cosolvent | Et-LGK | PGMME | EGMBE | DPGMME |
| Required amount of cosolvent to obtain clear solution, vol. % | | | | |
| 90/10 Water/IBIB ratio | 52.0 | 61.5 | 54.5 | 62.5 |
| 75/25 Water/IBIB ratio | 62.6 | 61.4 | 61.1 | 60.0 |
| 50/50 Water/IBIB ratio | 66.1 | 58.3 | 62.5 | 58.9 |
| 25/75 Water/IBIB ratio | 63.0 | 49.1 | 59.4 | 53.3 |
| 10/90 Water/IBIB ratio | 57.7 | 37.5 | 51.6 | 54.5 |

| | | | | |
|---|---|---|---|---|
| *Not an example of the invention. Ratios are by volume. PGMME is propylene glycol monomethyl ether. EGMBE is ethylene glycol monobutyl ether. DPGMME is dipropylene glycol monomethyl ether. | | | | |

Et-LGK is shown to have a similar ability to compatibilize water and IBIB as do each of the comparative cosolvents.

### Example 6 and Comparative Sample D-F - Castor Oil Solubilization

Mixtures of water and castor oil ChemistryStore.com; Cayce, South Carolina) are prepared at 10/90, 25/75, 50/50, 75/25 and 90/10 volume ratios. All form immiscible mixtures. To assess the ability of Et-LGK to solubilize castor oil into water, Et-LGK is added to each of these mixtures with vigorous stirring in a 20 mL scintillation vial at about 25°C until a clear solution is obtained. The Et- LGK concentration (in volume %) needed in each case to form a clear solution is reported in Table 4.

For comparison, the abilities of propylene glycol methyl ether (Acros Organics, a division of Thermo Fisher Scientific; Waltham, Massachusetts), ethylene glycol monobutyl ether (Sigma-Aldrich; St. Louis, Missouri) and dipropylene glycol methyl ether (Sigma-Aldrich; St. Louis, Missouri) to form a clear solution with water and castor oil are evaluated the same way. The concentrations of these comparative solvents (in volume %) needed in each case to form a clear solution is reported in Table 4 as Comparative Samples D, E and F, respectively.

**Table 4**

| | Example or Comparative Sample | | | |
|---|---|---|---|---|
| | 6 | D* | E* | F* |
| Cosolvent | Et-LGK | PGMME | EGMBE | DPGMME |
| Required amount of cosolvent to obtain clear solution, vol.-% | | | | |
| 90/10 Water/castor oil ratio | 84.1 | 83.3 | 61.0 | 80.6 |
| 75/25 Water/castor oil ratio | 84.7 | 81.3 | 64.1 | 78.9 |
| 50/50 Water/castor oil ratio | 80.3 | 76.2 | 60.5 | 73.5 |
| 25/75 Water/castor oil ratio | 72.3 | 65.0 | 54.8 | 65.4 |
| 10/90 Water/castor oil ratio | 63.4 | 60.5 | 44.4 | 55.9 |

| | | | | |
|---|---|---|---|---|
| *Not an example of the invention. Ratios are by volume. PGMME is propylene glycol monomethyl ether. EGMBE is ethylene glycol monobutyl ether. DPGMME is dipropylene glycol monomethyl ether. | | | | |

Et-LGK is shown to have a similar ability to compatibilize water and castor oil as do each of the comparative cosolvents.

### Example 7 and Comparative Examples G-I - Soya Methyl Esters Solubilization

Mixtures of water and soya methyl esters (Stepan Chemical, Northfield, Illinois) are prepared at 10/90, 25/75, 50/50, 75/25 and 90/10 volume ratios. All form immiscible mixtures. To assess the ability of Et-LGK to solubilize soya methyl esters into water, Et-LGK is added to each of these mixtures with vigorous stirring in a 20 mL scintillation vial at about 25°C until a clear solution is obtained. The Et-LGK concentration (in volume %) needed in each case to form a clear solution is reported in Table 5.

For comparison, the abilities of propylene glycol methyl ether (Acros Organics, a division of Thermo Fisher Scientific; Waltham, Massachusetts), ethylene glycol monobutyl ether (Sigma -Al drich; St. Louis, Missouri) and dipropylene glycol methyl ether (Sigma-Aldrich; St. Louis, Missouri) to form a clear solution with water and soya methyl esters are evaluated the same way. The concentrations of these comparative solvents (in volume %) needed in each case to form a clear solution is reported in Table 5 as Comparative Samples G, H and I, respectively.

**Table 5**

| | Example or Comparative Sample | | | |
|---|---|---|---|---|
| | 7 | G* | H* | I* |
| Cosolvent | Et-LGK | PGMME | EGMBE | DPGMME |
| Required amount of cosolvent to obtain clear solution, vol.-% | | | | |
| 90/10 Water/soya methyl ester ratio | 80.0 | 77.3 | 47.4 | 78.3 |
| 75/25 Water/soya methyl ester ratio | 85.4 | 80.6 | 54.1 | 78.3 |
| 50/50 Water/soya methyl ester ratio | 81.8 | 77.4 | 54.5 | 73.6 |
| 25/75 Water/soya methyl ester ratio | 76.7 | 67.1 | 50.9 | 62.6 |
| 10/90 Water/soya methyl ester ratio | 67.0 | 50.8 | 44.4 | 48.3 |

| | | | | |
|---|---|---|---|---|
| *Not an example of the invention. Ratios are by volume. PGMME is propylene glycol monomethyl ether. EGMBE is ethylene glycol monobutyl ether. DPGMME is dipropylene glycol monomethyl ether. | | | | |

As seen from the data in Table 5, Et-LGK and the comparative cosolvents all can compatibilize water and soya methyl esters oil.

### Example 8 and Comparative Examples J-L-2,2,4-Trimethyl-I,3-Pentanediol(2-methylpropanoate) Solubilization

Mixtures of water and 2,2,4-trimethyl-I,3-pentanediol (2-methyl propanoate) ("Texanol") (Sigma-Aldrich; St. Louis, Missouri) are prepared at 10/90, 25/75, 50/50, 75/25 and 90/10 volume ratios. All form immiscible mixtures. To assess the ability of Et-LGK to solubilize Texanol into water, Et-LGK is added to each of these mixtures with vigorous stirring in a 20 mL scintillation vial at about 25°C until a clear solution is obtained. The Et-LGK concentration (in volume%) needed in each case to form a clear solution is reported in Table 6.

For comparison, the abilities of propylene glycol methyl ether (Acros Organics, a division of Thermo Fisher Scientific; Waltham, Massachusetts), ethylene glycol monobutyl ether (Sigma-Aldrich; St. Louis, Missouri) and dipropylene glycol methyl ether (Sigma-Aldrich; St. Louis, Missouri) to form a clear solution with water and Texanol are evaluated the same way. The concentrations of these comparative solvents (in volume %) needed in each case to form a clear solution is reported in Table 6 as Comparative Samples G, H and I, respectively.

**Table 6**

| | Example or Comparative Sample | | | |
|---|---|---|---|---|
| | 8 | J* | K* | L* |
| Cosolvent | Et-LGK | PGMME | EGMBE | DPGMME |
| Required amount of cosolvent to obtain clear solution, vol.-% | | | | |
| 90/10 Water/Texanol ratio | 49.2 | 52.4 | 48.3 | 51.6 |
| 75/25 Water/Texanol ratio | 58.8 | 51.7 | 59.4 | 54.1 |
| 50/50 Water/Texatiol ratio | 61.0 | 49.2 | 62.5 | 55.2 |
| 25/75 Water/Texanol ratio | 59.4 | 41.7 | 53.7 | 50.9 |
| 10/90 Water/Texanol ratio | 49.2 | 30.2 | 45.5 | 44.4 |

| | | | | |
|---|---|---|---|---|
| *Not an example of the invention. Ratios are by volume. PGMME is propylene glycol monomethyl ether. EGMBE is ethylene glycol monobutyl ether. DPGMME is dipropylene glycol monomethyl ether. | | | | |

As seen from the data in Table 6, Et-LGK and the comparative cosolvents all can compatibilize water and Texanol.

### Examples 9 and 9A and Comparative Sample M - Et-LGK and Et-LPK in Cleaner Formulations

Cleaner formulations 9 and 9A are prepared by combining the following components in a 20 ml scintillation vial: 4 parts solvent (Example 9 - Et-LGK, and Example 9A - Et-LPK); 4.5 parts nonionic surfactant (Biosoft N91-6, Stepan Co., Northfield, Illinois); 0.5 parts sodium citrate dihydrate (Fisher Scientific, a division of Thermo Fisher Scientific; Waltham, Massachusetts), and the balance of the formulation being water. Comparative Sample M is prepared by replacing the solvent described in Examples 9 and 9A with additional water. Examples 9 and 9A and Comparative Sample M are heated separately with vigorous stirring, and visually observed to record the temperature at which the formulations turn cloudy. The cleaner formulation of Example 9 turns cloudy at about 48°C. The results obtained with Example 9 are similar to results that are obtained when the Et-LGK is replaced on a weight-for-weight basis with propylene glycol monomethyl ether, ethanol and ethylene glycol n-butyl ether. Example 9A turns cloudy at slightly above room temperature, which is consistent with the lower solubility of Et-LPK in water.

A vegetable oil-based soil composition (0.5 - 1.0 g) is prepared according to CSPA DCC- 17, "Greasy Soil Test Method for Evaluating Spray-and-Wipe cleaners Used on Hard, Non-Glossy Surfaces". The soil composition is applied to a 5 inch x 7 inch (12.5 cm X 15.5 cm) white porcelain on stainless steel tile. The soiled tiles are placed vertically in an oven at 150°C for 2 hours and aged overnight at room temperature. Cleaning tests are performed on a Gardner Linear Washability Tester (Byk-Gardner USA; Columbia, Maryland) with 200 g (gram) weights residing on the sponge attachment (ASTM D 2486). Examples 9, 9A and Comparative Sample M are separately evaluated. The tiles are washed for 25 cycles or until the tiles are > 98% clean, whichever occurred first.

The soiled tiles exposed to Example 9A are determined to be clean in only 12 cycles. The soiled tiles exposed to Example 9 show 85% soil removal in 25 cycles, whereas Comparative Sample M removes only 80 % of the soil after 25 cycles.

### Examples 10-15 - Cleaner Stability Evaluation

Cleaner Examples 10-12 are prepared by blending the following components: 4 parts of Et-LGK, 4.5 parts of nonionic surfactant (Biosoft N91-6, Stepan Co., Northfield, IL), 0.5 parts of an anionic surfactant, 0.5 parts of sodium citrate dihydrate (Fisher Scientific, a division of Thermo Fisher Scientific; Waltham, Massachusetts), and the balance of the formulation as water. In Example 10, the anionic surfactant is dodecyl benzene sulfonic acid (Acros Organics; Waltham, Massachusetts); in Example 11, the anionic surfactant is BioTerge PAS-8S (Stepan Co., Northfield, Illinois), and in Example 12, the surfactant is Dowfax CIO-L (Dow Chemical Company, Midland, Michigan). The cleaner formulations of Examples 10-12 are heated separately with vigorous stirring, and visually observed to determine the temperature at which the solutions become cloudy. Formulations 10-12 become cloudy at 72 °C, 55 °C and 60 °C, respectively. Similar results are observed when dipropylene glycol monomethyl ether or ethylene glycol monobutyl ether is substituted for the Et- LGK on a weight-for- weight basis.

Examples 13-15 are prepared and tested in the general manner described with respect to Examples 10-12 above, except the nonionic surfactant is Biosoft N25-7 (Stepan Co., Northfield, IL) in all cases. The cleaner formulations of Examples 13-15 are visually observed to become cloudy at 80 °C, 54 °C and 67°C, respectively. When dipropylene glycol monomethyl ether or ethylene glycol monobutyl ether is substituted for the Et-LGK in Examples 13-15 on a weight- for-weight basis, similar or lower cloud points are obtained, indicating that Et- LGK performs equivalently or better than those conventional solvents in these formulations in terms of solubilizing the nonionic surfactant.

### Reference Examples 16-17 and Comparative Examples N and O-Soft Surface Cleaner Formulations (Laundry Pre-Spotter)

Examples 16 and 17 are prepared by blending the following ingredients indicated in Table 7.

**Table 7**

| Component | Weight % | |
|---|---|---|
| | Example 16 | Example 17 |
| Water | 44.5 | 90.2 |
| Et-LGK | 20.0 | 0 |
| Et-LPK | 0 | 5.6 |
| Benzyl alcohol (Sigma-Aldrich; St. Louis, Missouri) | 8.0 | 0 |
| Ethylene glycol monobutyl ether (Sigma-Aldrich; St. Louis, Missouri) | 4.0 | 0 |
| Methyl soyate (Stepan Co.; Northfield, Illinois) | 8.0 | 0 |
| Tall oil fatty acids (Arizona Chemical; Panama City, Florida) | 10.0 | 0 |
| Potassium hydroxide, 45% aq. (Fisher Scientific, Waltham, Massachusetts) | 5.5 | 0 |
| Nonionic surfactant (Biosoft N25-7) | 0 | 1.9 |
| Nonionic surfactant (Biosoft N25-3) | 0 | 1.9 |
| Ethanol (Acros Organics; Waltham, Massachusetts) | 0 | 0.4 |

Separate 3" X 4" (7.5 cm X 10 cm) cotton/polyester swatches (Scientific Services S/D Inc.; Sparrow Bush, New York) are pre-stained with the following stains (all stains listed from Scientific Services S/D Inc.; Sparrow Bush, New York): ball point ink (Part No. 7435WRL, Lot No. 1345), used motor oil (Part No. 7435WRL, Lot No. 1912), lipstick (Part No. 7435WRL, Lot No. 1351), red wine (Part No. 7435WRL, Lot No. 1612), coffee (Part No. 7435WRL, Lot No. 1910) and blood (Part No. PEDP 7435WRL, Lot No. 1457). Duplicate samples of each swatch are treated with 2 mL of either Example 16 or Example 17, and subsequently washed 2-4 hours later in a commercial washing machine. The washing step is performed on a warm/cold setting and dried on a permanent press setting with three white cotton towels included as a ballast. Triplicate samples are analyzed for each test. The washed and dried swatches are visually inspected individually by five panelists, and rated for cleanliness based on a 1 to 5 (1 being poor and 5 being best) scale. The laundry pre-spotter performance test is based on CSPA DCC-II "Home Laundering Pre-Wash Spotter Stain Remover." Comparative Example N is conducted similarly to Examples 16-17, with the exception that a commercial pre-spotting formulation (Shout™; SC Johnson; Racine, Wisconsin) was used. Comparative Example O is the result of a similar cleaning process in which no pre-spotting formulation is present. Results are as indicated in Table 8.

**Table 8**

| | **Comparative Sample N*** | **Example 16** | **Example 17** | **Comparative Sample O*** |
|---|---|---|---|---|
| **Ratings** | | | | |
| Pen Ink | 2.8 | 4.0 | 3.0 | 1.7 |
| Motor Oil | 4.0 | 4.1 | 3.0 | 1.1 |
| Lipstick | 3.0 | 3.8 | 2.0 | 1.7 |
| Grass | 4.3 | 3.8 | 2.7 | 1.6 |
| Red wine | 3.4 | 2.8 | 4.3 | 2.5 |
| Coffee | 4.0 | 3.1 | 4.2 | 3.0 |
| Blood | 2.8 | 2.1 | 1.9 | 1.8 |
| Grape Juice | 3.8 | 3.3 | 3.5 | 2.3 |

Example 16 outperforms Comparative Samples N and O for ink and lipstick stains, and Example 17 performs very well for red wine and coffee stains. However, Comparative Sample N contains proteolytic enzymes, which were not present in Examples 16-17 or in Comparative Sample O.

### Reference Examples 18 and 19 and Comparative Samples P and Q: Concentrated Laundry Detergent Formulations

Examples 18 and 19 and Comparative Samples P and Q are prepared by combining the components listed in Table 9 in a 20 ml borosilicate glass scintillation vial and shaking by hand. Et-LGK replaces some of the water in each of Examples 18 and 19. All four formulations are clear, homogeneous solutions at room temperature.

**Table 9**

| **Comparative Sample P** | **Parts by Weight** | **Example 18** | **Parts by Weight** |
|---|---|---|---|
| Sodium chloride Nonionic surfactant: | 1.0 | Sodium chloride Nonionic surfactant: | 1.0 |
| BioSoft N25-7 Anionic surfactant: | 12.0 | BioSoft N25-7 Anionic surfactant: | 12.0 |
| Biosoft D-40 | 20.0 | Biosoft D-40 | 20.0 |
| Et-LGK | 0.0 | ET-LGK | 5.0 |
| Water | 67.0 | Water | 62.0 |
| | | | |

| **Comparative Sample Q** | **Parts by Weight** | **Example 19** | **Parts by Weight** |
|---|---|---|---|
| Sodium chloride Nonionic surfactant: | 1.0 | Sodium chloride Nonionic surfactant: | 1.0 |
| BioSoft N25-7 Anionic surfactant: | 24.0 | BioSoft N25-7 Anionic surfactant: | 24.0 |
| Biosoft D-40 | 40.0 | Biosoft D-40 | 40.0 |
| Et-LGK | 0.0 | Et-LGK | 5.0 |
| Water | 35.0 | Water | 30.0 |

Viscosities of Examples 18 and 19 and Comparative Samples P and Q are measured on a Brookfield DV-II+ Pro viscometer with 518 spindle at 25°C; results are listed in cP in Table 10.

**Table 10**

| Sample | RPM | Viscosity (cP) |
|---|---|---|
| Comparative Sample P | 20 | 406 |
| Comparative Sample Q | 20 | 337 |
| Example 18 | 100 | 37 |
| Example 19 | 20 | 200 |

As seen from the data in Table 10, the presence of Et-LGK leads to a significant reduction in the viscosity of the formulation. Examples 18-19 and Comparative Samples P and Q are placed in a refrigerator at 2°C for 1 hour. Comparative Sample Q becomes cloudy after 1 hour and all other solutions remain clear. After re-heating in a 50°C oven, all solutions are clear and homogeneous.

Examples 18-19 and Comparative Samples P and Q are placed in a freezer at -16°C for one hour, which freezes the solutions. All of the formulations are then allowed to thaw at room temperature. Comparative Sample Q remains cloudy after thawing; all remaining formulations return to clear, homogenous solutions. Examples 18 and 19 and Comparative Sample P are then subjected to two more freeze-thaw cycles in the same manner, after which Examples 18 and 19 and Comparative Sample P remain clear. These results indicate that the addition of Et-LGK to a concentrated laundry detergent formulation provides for a temperature-stable laundry detergent.

### Reference Examples 20-21 - Concentrated Cold Water Laundry Detergent Formulations

Examples 20-21 are prepared similar similarly to that described in US Patent Publication No. 2010/0041577 (Metalucca, Inc.). Formulations are listed in Table 11. The listed components are mixed in a 20 ml borosilicate glass scintillation vial, heated to 50°C for 15 minutes, and shaken mechanically overnight at room temperature. Both formulations are clear, homogeneous solutions. Viscosities of the above formulations are measured on a Brookfield DV- 11+ Pro viscometer with 518 spindle. Results are indicated in Table 11.

**Table 11**

| **Ingredient** | **Example 20** | **Example 21** |
|---|---|---|
| Nonionic surfactant: BioSoft N25-7 | 41.0 | 41.0 |
| Anionic surfactant: BioSoft N-300 (TEA/DDBSA, 60% soln.) | 31.7 | 31.7 |
| Et-LGK | 7.0 | 5.0 |
| Propylene Glycol | 0 | 7.0 |
| Water | 20.3 | 15.2 |
| **Viscosity, cP** | 214 | 173 |

The viscosities of these solutions are significantly lower than like solutions that are prepared without the alkyl ketal ester.

Examples 20 and 21 remain homogeneous after being placed in a 2°C refrigerator overnight, and remain homogeneous after three freeze-thaw cycles as described with respect to Examples 18 and 19.

### Reference Example 22 and Comparative Sample R - Aqueous Pot and Pan Degreaser Formulations

Example 22 and Comparative Sample R are prepared by combining the ingredients listed in Table 12 into a 20 ml borosilicate glass scintillation vial and shaking by hand. Both formulations are clear, homogeneous solutions.

**Table 12**

| **Ingredient** | **Comp. Sample R** | **Example 22** |
|---|---|---|
| Water | 70.5 | 70.5 |
| Monoethanolamine | 0.5 | 0.5 |
| Potassium carbonate | 2.0 | 2.0 |
| Sodium dihydrogen citrate | 2.0 | 2.0 |
| Anionic surfactant: Stepanate SCS-93 (93% Sodium cumene sulfonate) | 5.0 | 5.0 |
| Anionic surfactant: Biosoft D-40 (40% Sodium | | |
| DDBS) | 20.0 | 20.0 |
| Diethylene glycol butyl ether | 5.0 | 0.0 |
| Et-LPK | 0.0 | 5.0 |

Example 22 and Comparative Sample R each are heated to 60°C on a hotplate. Both formulations remain clear homogeneous solutions at 60°C, indicating both formulations have cloud points in excess of 60°C. Both solutions remain homogeneous after cooling for one hour in 2°C refrigerator. These results indicate that the alkyl ketal ester can replace the diethylene glycol butyl ether in a degreaser formulation.

### Example 23 - Aqueous Dish Gel Formulation

Example 23 is prepared from ingredients as indicated in Table 13.

**Table 13**

| **Ingredient** | **Amount,** % |
|---|---|
| Sodium hydroxide, 50% solution | 1.0 |
| Anionic surfactant: Biosoft D-40 (40% Sodium DDBS) | 20.0 |
| Xanthan Gum | 1.0 |
| Potassium Carbonate | 20.0 |
| Et-LGK | 5.0 |
| Water | 53.0 |

The NaOH is added to the water to a 2 oz polypropylene jar and heated to 50°C in an oven for 1 hour. The heated solution is stirred on a heated stir plate, and the xanthan gum is added over 2-3 minutes. The surfactant and the Et-LGK are sequentially added to the stirring solution. The heat is removed and the potassium carbonate is added to the solution with continued stirring at room temperature for 5 minutes. The vial is removed from the stir plate. The product has a translucent gray appearance. It is viscous but pourable. After sitting at room temperature for five days, this product thickens to a white gel that does not deflect when tipped to one side.

When Example 23 is duplicated, replacing the Et-LGK with water, the product is too viscous to pour. However, after five days, it forms a weak gel that cannot hold its shape when tipped to one side. The Et-LKG thus produces a firmer, superior gelled product.

### Reference Examples 24-26 - Baby Shampoo Formulations

Shampoo formulations 24-26 are prepared from the ingredients listed in Table 14.

**Table 14**

| Ingredient | Example 24 | Example 25 | Example 26 |
|---|---|---|---|
| DI Water | 74 | 85 | 84 |
| Xanthan Gum | 1 | 1 | 1 |
| Sulfosuccinate | 10 | 6 | 6 |
| Cocobetaine | 5 | 3 | 3 |
| Polyglucose | 4 | 2 | 2 |
| PEG-7 Glyceryl Cocoate | 0 | 0 | 0 |
| Et-LGK | 5 | 0 | 2 |
| Et-LPK | 0 | 3 | 2 |
| Parahen-DU | 1 | 1 | 1 |

All of these formulations are clear and homogeneous.

### Reference Examples 27-29 - Pearlizing Mild Shampoos

Examples 27-29 are prepared from the ingredients indicated in Table 15.

**Table 15**

| Ingredient | Example 27 | Example 28 | Example 29 |
|---|---|---|---|
| Phase A | | | |
| DI Water | 45 | 45 | 46 |
| Guar Gum | 1 | 1 | 1 |
| Glycerol | 0 | 2 | 0 |
| Et-LGK | 4 | 2 | 0 |

| Phase B | | | |
|---|---|---|---|
| Sodium Lauryl sulfate (25% in water) | 30 | 30 | 26 |
| Coco betaine | 10 | 10 | 10 |
| Polyglucose | 4 | 4 | 4 |
| Glycerol stearate IP | 4 | 4 | 4 |
| Et-LPK | 0 | 0 | 2 |
| | | | |
| PEG-150 Distearate | 0 | 0 | 4 |

| Phase C | | | |
|---|---|---|---|
| Pro Vitamin B5 | 0 | 1 | 1 |
| Phenoxyethanol SA | 0 | 2 | 2 |

All of these formulations are white in color and homogeneous.

### Reference Examples 30-31-Curly Hair Shampoo Formulations

Examples 30-31 are prepared from the ingredients indicated in Table 16.

**Table 16**

| Ingredient | Example 30 | Example 31 |
|---|---|---|
| Phase A | | |
| DI water | 43 | 43 |
| Polyquaternium-10 | 1 | 1 |

| Phase B | | |
|---|---|---|
| Sodium lauryl sulfate (25% in water) | 32 | 32 |
| Coco betaine | 16 | 16 |
| Quaternium-87 | 3 | 3 |
| PEG-150 distearate | 2 | 2 |
| PEG-7 Glyceryl cocoate | 0 | 0 |
| Et-LGK | 2 | 0 |
| Et-LPK | 0 | 2 |
| Cyclo-dimethicone | 1 | 1 |

| Phase C | | |
|---|---|---|
| Paraben-DU | 1 | 1 |

Examples 30 and 31 are opaque, as is expected, and homogeneous.

### Reference Examples 32-33- Green Tea Body Wash Formulations

Examples 32 and 33 are prepared from the ingredients listed in Table 17.

**Table 17**

| Ingredient | Example 32 | Example 33 |
|---|---|---|
| Phase A | | |
| Green Tea Infusion | 56 | 56 |
| Guar Gum | 1 | 1 |
| EDTA | 0.2 | 0.2 |

| Phase B | | |
|---|---|---|
| Sulfosuccinate | 22 | 22 |
| Coco betaine | 10 | 10 |
| Polyglucose | 5 | 5 |
| PEG-150 distearate | 3 | 3 |
| PEG-7 glyceryl cocoate | 0 | 0 |
| Et-LGK | 2 | 0 |
| Et-LPK | 0 | 2 |

| Phase C | | |
|---|---|---|
| Paraben DU | 1 | 1 |

### Reference Examples 34-35 - Shampoo Formulations

Examples 34 and 35 are prepared from the ingredients listed in Table 18.

**Table 18**

| Ingredient | Example 34 | Example 35 |
|---|---|---|
| Phase A | | |
| Sodium lauryl sulfate (25% in water) | 62 | 62 |
| Sodium lauroyl sarcosinate (10% in water) | 25 | 25 |
| Polyquaternium-10 | 1 | 1 |

| Phase B | | |
|---|---|---|
| PEG-150 Distearate | 4 | 4 |
| Coco Betaine | 5 | 5 |
| Et-LGK | 2 | 0 |
| Et-LPK | 0 | 2 |

| Phase C | | |
|---|---|---|
| Paraben-DU | 1 | 1 |

Examples 34 and 35 are both clear and homogeneous.

### Reference Examples 36 and 37 - Shower Body Wash Formulations

Examples 36 and 37 are prepared from the ingredients indicated in Table 19.

**Table 19**

| Ingredient | Ex. 36 | Ex. 37 |
|---|---|---|
| Phase A | | |
| Distilled water | 52 | 48.4 |
| Et-LGK | 2 | 0 |
| Glycerol | 2 | 2 |
| EDTA | 0.2 | 0.2 |

| Phase B | | |
|---|---|---|
| Sodium lauryl sulfate (25% in water) | 20 | 20 |
| Et-LPK | 0 | 2 |
| Sulfosuccinate | 10 | 10 |
| Coco Betaine | 8 | 8 |
| PEG-150 distearate | 2 | 6 |
| PEG-7 Glyceryl stearate | 2 | 2 |

| Phase C | | |
|---|---|---|
| Phenoxyethanol | 1 | 1 |

### Reference Examples 38 and 39 - Hard Surface Cleaner

The following formulations are made by preparing Phase I and Phase II in separate 20 ml borosilicate glass scintillation vial, then slowly pouring Phase I into Phase II while stirring. The ingredients are listed in Table 20.

**Table 20**

| **Ingredient** | **Parts by Weight** | |
|---|---|---|
| | Ex. 38 | Ex. 39 |
| **Phase I** | | |
| Isopropanol | 10.0 | 0 |
| Oleic Acid | 9.0 | 9.0 |
| Nonionic Surfactant (Biosoft N91-6) | 5.0 | 5.0 |
| Et-LPK | 12.0 | 0 |
| Et-LGK | 0 | 10.0 |
| d-limonene | 0 | 12.0 |

| **Phase II** | | |
|---|---|---|
| Potassium hydroxide | 1.75 | 1.75 |
| Water | 62.25 | 62.25 |

Example 38 is a clear, low viscosity solution whereas Example 39 is a cloudy, slightly viscous emulsions which remains stable overnight at room temperature.

### Reference Examples 40 and 41 - Hard Surface Cleaner Concentrate

The following formulations are made by preparing Phase I and Phase II in separate 20 ml borosilicate glass scintillation vial, then slowly pouring Phase I into Phase II while stirring. The ingredients are listed in Table 21.

**Table 21**

| **Ingredient** | **Parts by Weight** | |
|---|---|---|
| | Ex. 40 | Ex. 41 |
| **Phase I** | | |
| Isopropanol | 1.5 | 0 |
| Oleic Acid | 1.4 | 1.4 |
| Nonionic Surfactant (Biosoft N91-6) | 0.8 | 0.8 |
| Et-LPK | 1.8 | 0 |
| Et-LGK | 0 | 1.5 |
| d-limonene | 0 | 1.8 |

| **Phase II** | | |
|---|---|---|
| Potassium hydroxide | 0.26 | 0.26 |
| Water | 0.8 | 0.8 |

Examples 40 and 41 are both clear, low viscosity solutions. When diluted with 8.5 parts of water, Example 40 remains clear whereas Example 41 becomes cloudy and slightly viscous.

### Example 42 - Soap Scum Remover

Example 42 is made by mixing 5 parts of tetrasodium EDTA, 10 parts of an anionic surfactant (Biosoft D-40), 5 parts of Et-LGK and 80 parts of water. A clear, homogeneous solution results.

### Reference Example 43 - Surfactant-free makeup remover

Example 43 is made by mixing 5 parts of Et-LPK, 19.5 parts of Et-LGK and 5 parts of water. The product is a clear homogeneous makeup remover formulation without a surfactant.

### Reference Examples 44-48 - Eye Makeup Remover Formulations with Surfactant

Examples 44-48 are prepared from the listing of ingredients in Table 22.

The ingredients of Examples 44-48 are added to a beaker in the order listed in Table 23 and mixed thoroughly.

**Table 22**

| Component | Parts by Weight | | | | |
|---|---|---|---|---|---|
| | 44 | 45 | 46 | 47 | 48 |
| Ethyl-LPK | 27.3 | 27.3 | 23.1 | 20.0 | 17.6 |
| 1,3-propane diol | 36.4 | 0.0 | 0.0 | 0.0 | 0.0 |
| 1,2-Propane diol | 0.0 | 36.4 | 30.8 | 26.7 | 23.5 |
| Ethyl-LGK | 27.3 | 27.3 | 23.1 | 20. | 17.6 |
| 10% Sodium Lauryl Sarcosinate in water | 9.1 | 9.1 | 23.1 | 33.3 | 41.2 |

| | | | | | |
|---|---|---|---|---|---|
| *Not an example of the invention | | | | | |

Each of Examples 44-48 are clear homogeneous solutions.

## Claims

1. A cleaning formulation comprising
a) from 50-95 % by weight water;
b) from 0.1 to 10 weight percent of at least one alkyl ketal ester(s) and
c) from 1 to 10 weight percent of at least one surfactant, wherein component b) is one or more of 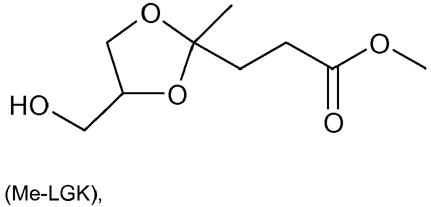 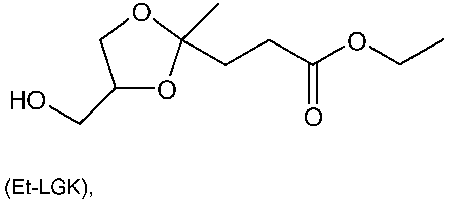 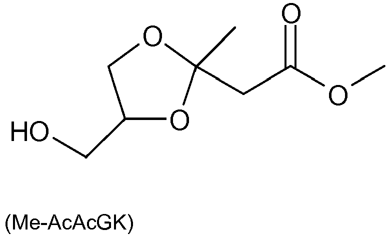 or

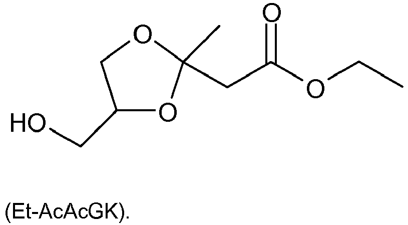

2. The cleaning formulation of claim 1, further comprising at least one of components d) to p), wherein d) to p) are:
d) one or more propellants;
e) one or more sparingly water-miscible organic solvent(s);
f) one or more highly water-miscible organic solvents;
g) one or more antimicrobials;
h) one or more builders or chelating agents;
i) one or more bleaches;
j) one or more pH control agent(s);
k) one or more colorants;
l) one or more inorganic water-soluble salts;
m) one or more viscosity thickeners;
n) one or more proteolytic enzymes;
o) one or more abrasives; and
p) one or more fragrances.

3. The cleaning formulation of claim 2 which contains up to 5% by weight of component e), up to 5% by weight of component f), up to 2% by weight of component g), up to 30% by weight of component h), up to 10% by weight of component (i), up to 5% by weight of component k), up to 10% by weight of component l), up to 10% by weight of component m) and up to 1% by weight of component n).

4. A method for cleaning a soiled substrate, comprising bringing a composition of any claims 1 to 3 into contact with a soiled substrate and then removing the composition cleaner together with removed soils.

## Patentansprüche

1. Reinigungsformulierung, die Folgendes umfasst:
a) von 50-95 Gewichts-% Wasser;
b) von 0,1 bis 10 Gewichtsprozent von mindestens einem Alkylketalester und
c) von 1 bis 10 Gewichtsprozent von mindestens einem Tensid, wobei Komponente b) eines oder mehrere von Folgenden ist: 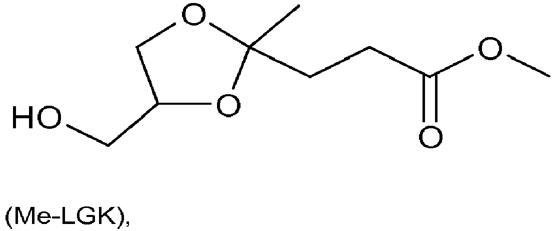 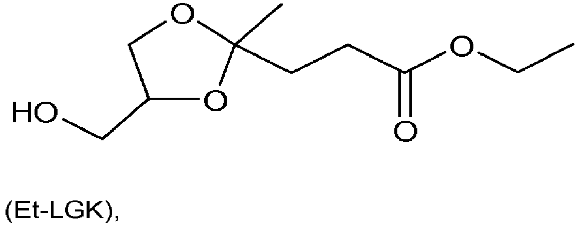 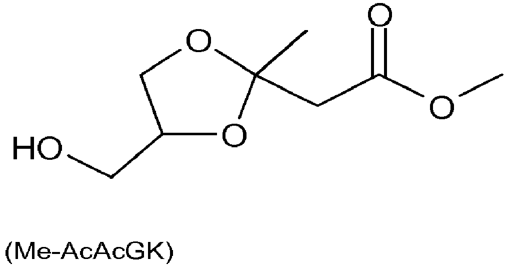 oder

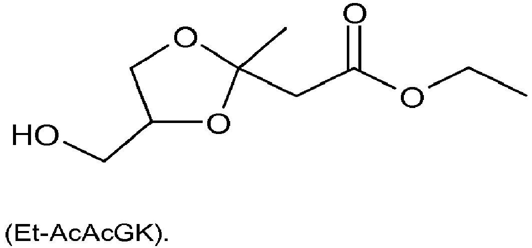

2. Reinigungsformulierung nach Anspruch 1, die weiter mindestens eine von Komponenten d) bis p) umfasst, wobei d) bis p) Folgendes sind:
d) ein oder mehrere Treibmittel;
e) ein oder mehrere schlecht mit Wasser mischbare, organische Lösungsmittel;
f) ein oder mehrere gut mit Wasser mischbare, organische Lösungsmittel;
g) ein oder mehrere antimikrobielle Mittel;
h) ein oder mehrere Builder oder Komplexbildner;
i) ein oder mehrere Bleichmittel;
j) ein oder mehrere pH-Kontrollmittel;
k) ein oder mehrere Farbstoffe;
l) ein oder mehrere anorganische wasserlösliche Salze;
m) ein oder mehrere Viskositätsverdickungsmittel;
n) ein oder mehrere proteolytische Enzyme;
o) ein oder mehrere Abrasivstoffe; und
p) ein oder mehrere Duftstoffe.

3. Reinigungsformulierung nach Anspruch 2, die Folgendes enthält: bis zu 5 Gewichts-% von Komponente e), bis zu 5 Gewichts-% von Komponente f), bis zu 2 Gewichts-% von Komponente g), bis zu 30 Gewichts-% von Komponente h), bis zu 10 Gewichts-% von Komponente (i), bis zu 5 Gewichts-% von Komponente k), bis zu 10 Gewichts-% von Komponente l), bis zu 10 Gewichts-% von Komponente m) und bis zu 1 Gewichts-% von Komponente n).

4. Verfahren für die Reinigung eines verschmutzten Substrats, das das Inkontaktbringen einer Zusammensetzung nach einem der Ansprüche 1 bis 3 mit einem verschmutzten Substrat und dann Entfernen des Zusammensetzungsreinigers zusammen mit entferntem Schmutz umfasst.

## Revendications

1. Formulation de nettoyage, comprenant
a) 50-95% en poids d'eau ;
b) de 0,1 à 10% en poids d'au moins un ester d'alkylcétal ; et
c) de 1 à 10% en poids d'au moins un agent tensioactif, où le composant b) est l'un ou plusieurs parmi : 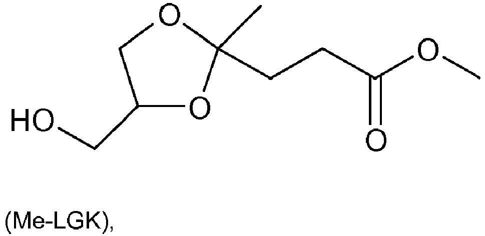 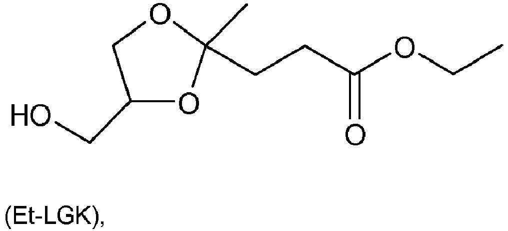 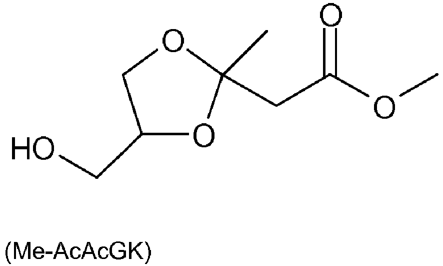 ou

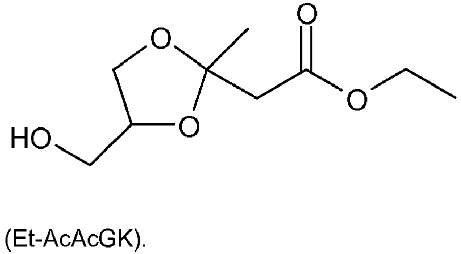

2. Formulation de nettoyage selon la revendication 1, comprenant en outre au moins l'un parmi les composants d) à p), où d) à p) sont :
d) un ou plusieurs propulseurs ;
e) un ou plusieurs solvants organiques peu miscibles avec l'eau ;
f) un ou plusieurs solvants organiques hautement miscibles avec l'eau ;
g) un ou plusieurs agents antimicrobiens ;
h) un ou plusieurs adjuvants ou agents chélatants ;
i) un ou plusieurs agents de blanchiment ;
j) un ou plusieurs agents de contrôle du pH ;
k) un ou plusieurs colorants ;
l) un ou plusieurs sels hydrosolubles inorganiques ;
m) un ou plusieurs épaississants de viscosité ;
n) une ou plusieurs enzymes protéolytiques ;
o) un ou plusieurs agents d'abrasion ; et
p) un ou plusieurs parfums.

3. Formulation de nettoyage selon la revendication 2, qui contient jusqu'à 5% en poids du composant e), jusqu'à 5% en poids du composant f), jusqu'à 2% en poids du composant g), jusqu'à 30% en poids du composant h), jusqu'à 10% en poids du composant i), jusqu'à 5% en poids du composant k), jusqu'à 10% en poids du composant l), jusqu'à 10% en poids du composant m), et jusqu'à 1% en poids du composant n).

4. Méthode de nettoyage d'un substrat souillé, comprenant la mise en contact d'une composition selon l'une quelconque des revendications 1 à 3 avec un substrat souillé, puis l'élimination du nettoyant en composition conjointement avec les souillures éliminées.
